# EUROPÄISCHE PATENTSCHRIFT

(11) **EP 1 406 888 B1**
(45) Veröffentlichungstag und Bekanntmachung des Hinweises auf die Patenterteilung: **24.05.2006**
(21) Anmeldenummer: 02743260.8
(22) Anmeldetag: 03.07.2002
(51) Int. Cl.: C07D 307/32, C07D 405/12, A61K 31/341, A61P 23/00

(54) **SUBSTITUIERTE Y-LACTONVERBINDUNGEN ALS NMDA-ANTAGONISTEN**
SUBSTITUTED Y-LACTONE COMPOUNDS SERVING AS NMDA ANTAGONISTS
COMPOSES Y-LACTONES SUBSTITUES EN TANT QU'ANTAGONISTES NMDA

(30) Priorität: 05.07.2001 DE 10132725
(43) Veröffentlichungstag der Anmeldung: 14.04.2004
(73) Patentinhaber: Grünenthal GmbH, 52078 Aachen (DE)
(72) Erfinder: MAUL, Corinna, 52066 Aachen (DE); PRZEWOSNY, Michael, 52062 Aachen (DE); ENGLBERGER, Werner, 52223 Stolberg (DE)
(74) Vertreter: Kutzenberger, Helga
(86) Internationale Anmeldenummer: PCT/EP2002/007380
(87) Internationale Veröffentlichungsnummer: WO 2003/004483

(56) Entgegenhaltungen:
- GONZALEZ, DAVID ET AL: "Chemoenzymic Synthesis of Unnatural Amino Acids via Modified Claisen Rearrangement of Glycine Enolates. Approach to Morphine Synthesis" JOURNAL OF ORGANIC CHEMISTRY (1997), 62(5), 1194-1195 , XP001097342
- PLUSQUELLEC, DANIEL: "Friedel-Crafts reactions of.gamma.-aryl-.gamma.-lactones" J. CHEM. RES., SYNOP. (1982), (2), 46-7 , XP001097650
- PLUSQUELLEC, DANIEL ET AL: "Reactions of glyoxylic acid derivatives with olefins: syntheses of.alpha.-substituted.gamma.-lactones" BULL. SOC. CHIM. FR. (1979), (9-10, PT. 2), 552-8 , XP001097998
- BEN-ISHAI, D. ET AL: "A new synthesis of amino acids. II. Amidoalkylation of olefins with glyoxylic acid derivatives" TETRAHEDRON (1977), 33(12), 1533-42 , XP001095785
- ALTMAN, JANINA ET AL: "Amino acid synthesis. II. Amidoalkylation of olefins with glyoxylic aci derivatives" TETRAHEDRON LETT. (1975), (43), 3737-40 , XP001097977
- FOSCOLOS G B ET AL: "SYNTHESIS AND PHARMACOLOGICAL STUDY OF SOME NEW BETA-(DIALKYLAMINOMETHYL)-GAMMA-BUTYROLACT ONES AND THEIR TETRAHYDROFURAN ANALOGUES" FARMACO, SOCIETA CHIMICA ITALIANA, PAVIA, IT, Bd. 1, Nr. 51, 1996, Seiten 19-26, XP000915050 ISSN: 0014-827X

## Beschreibung

Die vorliegende Erfindung betrifft substituierte γ-Lactonverbindungen, Verfahren zu deren Herstellung, Arzneimittel enthaltend diese Verbindungen sowie die Verwendung dieser Verbindungen zur Herstellung von Arzneimitteln.

Die Behandlung chronischer und nicht chronischer Schmerzzustände hat in der Medizin eine große Bedeutung. Es besteht ein weltweiter Bedarf an gut wirksamen Schmerztherapien für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist. Dies zeigt sich in der großen Anzahl von wissenschaftlichen Arbeiten, die auf dem Gebiet der angewandten Analgetik bzw. der Grundlagenforschung zur Nociception in letzter Zeit erschienen sind.

Klassische Opioide, wie beispielsweise Morphin, sind bei der Therapie starker bis stärkster Schmerzen gut wirksam. Ihr Einsatz wird jedoch durch die bekannten Nebenwirkungen, wie z.B. Atemdepression, Erbrechen, Sedierung, Obstipation und Toleranzentwicklung, limitiert. Außerdem sind sie bei neuropathischen oder inzidentiellen Schmerzen, unter denen insbesondere Tumorpatienten leiden, weniger wirksam.

Opioide entfalten ihre analgetische Wirkung durch Bindung an membranständige Rezeptoren, die zur Familie der sogenannten G-Proteingekoppelten Rezeptoren gehören. Die biochemische und pharmakologische Charakterisierung von Subtypen dieser Rezeptoren hat nun die Hoffnung geweckt, daß subtypenspezifische Opioide über ein anderes Wirkungs-/Nebenwirkungsprofil als z.B. Morphin verfügen. Weitere pharmakologische Untersuchungen haben inzwischen die Existenz mehrerer Subtypen dieser Opioidrezeptoren wahrscheinlich gemacht.

Daneben gibt es weitere Rezeptoren und lonenkanäle, die wesentlich an dem System der Schmerzentstehung und Schmerzweiterleitung beteiligt sind. Von besonderer Bedeutung ist dabei der NMDA-lonenkanal, über den ein wesentlicher Teil der Kommunikation von Synapsen abläuft. Durch diesen Kanal wird der Calcium-lonenaustausch zwischen einer neuronalen Zelle und ihrer Umgebung gesteuert.

Kenntnisse über die physiologische Bedeutung von lonenkanal-selektiven Substanzen sind durch die Entwicklung der patch-clamp-Technik gewonnen worden. So läßt sich eindeutig die Wirkung von NMDA-Antagonisten auf den Einfluß von Calcium-lonen in das Zellinnere nachweisen. Es stellte sich heraus, daß diese Substanzen über ein eigenständiges antinociceptives Potential verfügen, wie z.B. Ketamin. Wichtig ist, daß der Wirkmechanismus ein ganz anderer ist, wie beispielsweise bei den Opiaten, denn durch NMDA-Antagonisten wird direkt in den entscheidenden Calciumhaushalt der Zellen bei der Schmerzweiterleitung eingegriffen. Daher besteht erstmalig die Möglichkeit, auch die Behandlung von neuropathischen Schmerzformen erfolgreich durchzuführen.

Verschiedene NMDA-Antagonisten aus der Gruppe der Tetrahydrochinolinderivate wurden bereits in J. Med. Chem. (1992) 35, Seiten 1954-1968; J. Med. Chem. (1992) 35, Seiten 1942-1953 und Med. Chem. Res. (1991) 1; Seiten 64-73 sowie in der EP 0 386 839, WO 97/12879, WO 98/07704 und WO 98/42673 beschrieben.

In Farmaco, 51(1), 19-26 (1996) wird über die mögliche Aktivierung von NMDA-Rezeptoren durch Dihydrofuranon-Derivate berichtet.

Femer wurde eine Vielzahl von Indikationen angegeben, die einer Behandlung mit NMDA-Antagonisten zugänglich sind, unter anderen auch die Schmerztherapie.

In der Veröffentlichung Tetrahedron Letters 2000, 41, 9747-9751 wird die Verbindung 5-Phenyl-3-(2,4,6-trichlor-phenylamino)-5-methyl-dihydro-furan-2-on beschrieben.

Darüber hinaus besteht aber noch weiterer Bedarf an wirksamen NMDA-Antagonisten, die sich vorzugsweise zur Schmerzbekämpfung eignen.

Eine der vorliegenden Erfindung zugrundeliegende Aufgabe bestand daher darin, neue Verbindungen zur Verfügung zu stellen, die sich insbesondere als pharmazeutische Wirkstoffe in Arzneimitteln, vorzugsweise als Arzneimittel zur Schmerzbekämpfung, insbesondere für die Therapie von chronischen oder neuropathischen Schmerzen eignen. Des weiteren sollen sich diese Wirkstoffe auch zur Behandlung oder Prophylaxe von neurodegenerativen Erkrankungen, insbesondere von Morbus Alzheimer, Morbus Parkinson oder Morbus Huntington, von Migräne, Schlaganfall, cerebraler Ischämie, cerebralem Infarkt, Himödem, Schizophrenie, Psychosen bedingt durch erhöhten Aminosäurespiegel, AIDS-Demenz, Tourette-Syndrom, inflammatorischen und/oder allergischen Reaktionen, Depressionen, seelischen Erkrankungen, Epilepsie, Haminkontinenz, Juckreiz, Tinnitus aurium, Diarrhoe, zur Anxiolyse oder zur Anästhesie eignen.

Überraschenderweise wurde gefunden, daß substituierte γ-Lactonverbindungen der nachstehenden allgemeinen Formel I als NMDA-Antagonisten wirken, indem sie an der Glycin-Bindungsstelle des NMDA-Rezeptorkanals angreifen. Sie eignen sich daher zur Bekämpfung von Schmerz, insbesondere zur Bekämpfung von chronischem oder neuropathischem Schmerz, aber auch zur Behandlung oder Prophylaxe von neurodegenerativen Erkrankungen, insbesondere von Morbus Alzheimer, Morbus Parkinson oder Morbus Huntington, von Migräne, Schlaganfall, cerebraler Ischämie, cerebralem Infarkt, Himödem, Schizophrenie, Psychosen bedingt durch erhöhten Aminosäurespiegel, AIDS-Demenz, Tourette-Syndrom, inflammatorischen und/oder allergischen Reaktionen, Depressionen, seelischen Erkrankungen, Epilepsie, Haminkontinenz, Juckreiz, Tinnitus aurium, Diarrhoe, zur Anxiolyse oder zur Anästhesie.

Ein Gegenstand der vorliegenden Erfindung sind daher substituierte γ-Lactonverbindungen der allgemeinen Formel I 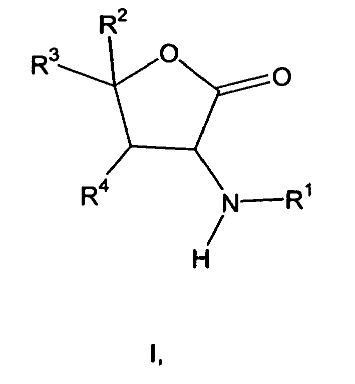 worin
R¹ für einen gegebenenfalls wenigstens einfach substituierten Aryl- oder Heteroaryl-Rest, bevorzugt für einen gegebenenfalls wenigstens einfach substituierten Aryl-Rest, steht,
R² für einen gegebenenfalls wenigstens einfach substituierten, gesättigten, verzweigten oder unverzweigten aliphatischen C₁₋₁₀-Rest oder für einen gegebenenfalls wenigstens einfach substituierten, zumindest teilweise ungesättigten, verzweigten oder unverzweigten aliphatischen C₂₋₁₀-Rest, vorzugsweise für einen gegebenenfalls wenigstens einfach substituierten, verzweigten oder unverzweigten C₁₋₆-Alkyl-Rest, steht,
R³ für einen gegebenenfalls wenigstens einfach substituierten Aryl-Rest steht
und
R⁴ für H steht,
oder
R³ und R⁴ zusammen für einen gegebenenfalls wenigstens einfach substituierten, gesättigten oder wenigstens einfach ungesättigten C₃₋₇-Rest stehen, mit der Maßgabe, daß der Rest R² in diesem Fall für einen gegebenenfalls wenigstens einfach substituierten Aryl-Rest, für einen gegebenenfalls wenigstens einfach substituierten, gesättigten, verzweigten oder unverzweigten aliphatischen C₁₋₁₀-Rest oder für einen gegebenenfalls wenigstens einfach substituierten, zumindest teilweise ungesättigten, verzweigten oder unverzweigten aliphatischen C₂₋₁₀-Rest steht,

in Form ihrer Racemate, Diastereomere oder Enantiomere als freie Base oder eines entsprechenden physiologisch verträglichen Salzes,
wobei die Verbindung der allgemeinen Formel I, worin R¹ für einen 2-,4-,6-Trichlorphenyl-Rest, R² für einen Methyl-Rest, R³ für einen Phenyl-Rest und R⁴ für H steht, ausgenommen ist.

Bevorzugt sind γ-Lactonverbindungen der allgemeinen Formel I, worin der Rest R³ für einen gegebenenfalls wenigstens einfach substituierten Aryl-Rest und der Rest R⁴ für H steht.

Die aliphatischen Reste können einfach oder mehrfach substituiert sein. Sofern die aliphatischen Reste mehr als einen Substituenten aufweisen, können diese gleich oder verschieden sein und sowohl an dem selben wie auch an verschiedenen Atomen des aliphatischen Restes gebunden sein.
Bevorzugt ist der aliphatische Rest ausgewählt aus der Gruppe bestehend aus gegebenenfalls wenigstens einfach substituiertem Methyl, Ethyl, Propyl, Isopropyl, n-Butyl, sek-Butyl, tert-Butyl, n-Pentyl, Neopentyl, n-Hexyl, n-Heptyl, n-Octyl, n-Nonyl, n-Decyl, Propenyl, Butenyl, Pentenyl, Hexenyl, Heptenyl, Octenyl, Propinyl, Butinyl, Pentinyl, Hexinyl, Heptinyl und Octinyl. Die Substituenten sind ausgewählt aus der Gruppe bestehend aus F, CI, Br, I, NH₂, SH und OH.

Die cycloaliphatischen Reste können einfach oder mehrfach substituiert sein. Sofern die cycloaliphatischen Reste mehr als einen Substituenten aufweisen, können diese gleich oder verschieden sein und sowohl an dem selben wie auch an verschiedenen Atomen des cycloaliphatischen Restes gebunden sein. Bevorzugt ist der cycloaliphatische Rest ein gegebenenfalls wenigstens einfach substituierter Cyclopropyl-, Cyclobutyl-, Cyclopentyl-, Cyclohexyl-, Cycloheptyl-, Cyclopentenyl-, Cyclohexenyl- oder Cycloheptenyl-Rest. Die Substituenten sind ausgewählt aus der Gruppe bestehend aus F, Cl, Br, I, NH₂, SH und OH.

Unter einem Aryl-Rest werden im Sinne der vorliegenden Erfindung auch solche aromatischen Kohlenwasserstoffreste verstanden, die mit einem gesättigten oder zumindest teilweise ungesättigten Kohlenwasserstoff-Ringsystem kondensiert sind.
Bevorzugt ist als Aryl-Rest ein gegebenenfalls wenigstens einfach substituierter Phenyl-, Naphthyl- oder Anthracenyl-Rest, besonders bevorzugt ein gegebenenfalls einfach substituierter Phenyl-Rest.

Sofern der Aryl-Rest mehr als einen Substituenten aufweist, können diese gleich oder verschieden sein. Die Substituenten sind ausgewählt aus der Gruppe bestehend aus F, Cl, Br, I, NH₂, SH, OH, CF₃, CN, NO₂, OR⁵, SR⁵, NR⁶R⁷ und unsubstituiertem oder wenigstens einfach mit F, Cl, Br, I, NH₂, SH, OH, CF₃, CN oder NO₂ substituiertem C₁₋₆-Alkyl, C₁₋₆-Alkoxy, C₂₋₈-Alkenyl, C₂₋₈-Alkinyl, Phenyl, Phenoxy oder Benzyloxy, wobei
R⁵ für H, einen C₁₋₁₀-Alkyl-Rest, einen unsubstituierten Aryl- oder Heteroaryl-Rest oder für einen unsubstituierten, über eine C₁₋₃-Alkylen-Gruppe gebundenen Aryl- oder Heteroaryl-Rest steht und
R⁶ und R⁷, gleich oder verschieden, für H, einen C₁₋₁₀-Alkyl-Rest, einen unsubstituierten Aryl- oder Heteroaryl-Rest oder für einen unsubstituierten, über eine C₁₋₃-Alkylen-Gruppe gebundenen Aryl- oder Heteroaryl-Rest stehen.

Unter einem Heteroaryl-Rest werden im Sinne der vorliegenden Erfindung auch solche heteroaromatischen Kohlenwasserstoffreste verstanden, die mit einem gesättigten oder zumindest teilweise ungesättigten Kohlenwasserstoff-Ringsystem kondensiert sind. Vorzugsweise enthält der Heteroaryl-Rest ein Heteroatom ausgewählt aus der Gruppe bestehend aus Schwefel, Stickstoff und Sauerstoff.

Bevorzugt ist als Heteroaryl-Rest ein gegebenenfalls wenigstens einfach substituierter Thiophenyl-, Furanyl-, Pyrrolyl-, Pyridinyl-, Pyrimidinyl-, Chinolinyl-, Isochinolinyl-, Phthalazinyl- oder Chinazolinyl-Rest.
Sofern der Heteroaryl-Rest mehr als einen Substituenten aufweist, können diese gleich oder verschieden sein. Die Substituenten sind ausgewählt aus der Gruppe bestehend aus F, Cl, Br, I, NH₂, SH, OH, CF₃, CN, NO₂, OR⁵, SR⁵, NR⁶R⁷ und unsubstituiertem oder wenigstens einfach mit F, Cl, Br, I, NH₂, SH, OH, CF₃, CN oder NO₂ substituiertem C₁₋₆-Alkyl, C₁₋₆-Alkoxy, C₂₋₈-Alkenyl, C₂₋₈-Alkinyl, Phenyl, Phenoxy oder Benzyloxy, wobei die Reste R⁵, R⁶ und R⁷ die oben angegebene Bedeutung haben.

Ganz besonders bevorzugt sind folgende substituierte γ-Lactonverbindungen:
3-(2-Chlor-4-fluor-phenylamino)-5-(4-fluor-phenyl)-5-methyl-dihydro-furan-2-on,
5-Methyl-3-(4-phenoxy-phenylamino)-5-phenyl-dihydro-furan-2-on,
3-(2-Chlor-phenylamino)-5-(4-fluor-phenyl)-5-methyl-dihydro-furan-2-on,
3-(4-Chlor-2-methyl-phenylamino)-5-methyl-5-phenyl-dihydro-furan-2-on,
3-(2,4-Dichlor-phenylamino)-5-(4-fluor-phenyl)-5-methyl-dihydro-furan-2-on,
3-(4-Chlor-3-trifluormethyl-phenylamino)-5-methyl-5-phenyl-dihydro-furan-2-on,
3-(2,3-Dichlor-phenylamino)-5-(4-fluor-phenyl)-5-methyl-dihydro-furan-2-on,
3-(4-lod-phenylamino)-5-methyl-5-phenyl-dihydro-furan-2-on,
3-(4-Chlor-2-fluor-phenylamino)-5-(4-fluor-phenyl)-5-methyl-dihydro-furan-2-on,
3-(2-Chlor-4-methyl-phenylamino)-5-methyl-5-phenyl-dihydro-furan-2-on,
3-(2-Chlor-4-methyl-phenylamino)-5-(4-fluor-phenyl)-5-methyl-dihydro-furan-2-on,
3-(3,5-Dichlor-phenylamino)-5-methyl-5-phenyl-dihydro-furan-2-on,
3-(3,5-Dichlor-phenylamino)-5-(4-fluor-phenyl)-5-methyl-dihydro-furan-2-on,
3-(4-Brom-2-chlor-phenylamino)-5-(4-fluor-phenyl)-5-methyl-dihydro-furan-2-on,
4-(5-Methyl-2-oxo-5-phenyl-tetrahydro-furan-3-ylamino)-benzonitril,
5-(4-Chlor-phenyl)-3-(4-iod-phenylamino)-5-methyl-dihydro-furan-2-on,
5-(4-Chlor-phenyl)-3-(2,4-dichlor-phenylamino)-5-methyl-dihydro-furan-2-on,
5-(4-Chlor-phenyl)-3-(2-chlor-phenylamino)-5-methyl-dihydro-furan-2-on,
3-(4-Chlor-2-methyl-phenylamino)-5-(4-chlor-phenyl)-5-methyl-dihydro-furan-2-on,
3-(2-Chlor-4-fluor-phenylamino)-5-(4-chlor-phenyl)-5-methyl-dihydro-furan-2-on,
3-(4-Chlor-2-fluor-phenylamino)-5-(4-chlor-phenyl)-5-methyl-dihydro-furan-2-on,
3-(2-Chlor-4-methyl-phenylamino)-5-(4-chlor-phenyl)-5-methyl-dihydro-furan-2-on,
5-(4-Chlor-phenyl)-3-(2,3-dichlor-phenylamino)-5-methyl-dihydro-furan-2-on,
3-(4-Brom-2-chlor-phenylamino)-5-(4-chlor-phenyl)-5-methyl-dihydro-furan-2-on,
5-(4-Chlor-phenyl)-3-(3,5-dichlor-phenylamino)-5-methyl-dihydro-furan-2-on,
3-(3,5-Dibrom-pyridin-2-ylamino)-5-methyl-5-phenyl-dihydro-furan-2-on,
5-(4-Chlor-phenyl)-3-(3,5-dichlor-pyridin-2-ylamino)-5-methyl-dihydro-furan-2-on,
5-(4-Chlor-phenyl)-5-methyl-3-(5-nitro-pyridin-2-ylamino)-dihydro-furan-2-on,
3-(3-Chlor-2-methyl-phenylamino)-5-(4-iod-phenyl)-5-methyl-dihydro-furan-2-on,
5-(4-Brom-phenyl)-3-(4-chlor-phenylamino)-5-methyl-dihydro-furan-2-on,
5-(3-Chlor-phenyl)-3-(4-chlor-phenylamino)-5-methyl-dihydro-furan-2-on,
3-(4-Chlor-phenylamino)-5-(4-iod-phenyl)-5-methyl-dihydro-furan-2-on,
5-(4-Brom-phenyl)-3-(2-iod-phenylamino)-5-methyl-dihydro-furan-2-on,
5-(3-Chlor-phenyl)-3-(2-iod-phenylamino)-5-methyl-dihydro-furan-2-on,
5-(4-lod-phenyl)-3-(2-iod-phenylamino)-5-methyl-dihydro-furan-2-on,
3-(2,4-Difluor-phenylamino)-5-methyl-5-naphthalin-1-yl-dihydro-furan-2-on,
5-(4-Brom-phenyl)-3-(4-iod-phenylamino)-5-methyf-dihydro-furan-2-on,
5-(3-Chlor-phenyl)-3-(4-iod-phenylamino)-5-methyl-dihydro-furan-2-on,
3-(4-lod-phenylamino)-5-methyl-5-naphthalin-1-yl-dihydro-furan-2-on,
5-(4-Brom-phenyl)-3-(3,5-dichlor-phenylamino)-5-methyl-dihydro-furan-2-on,
5-(3-Chlor-phenyl)-3-(3,5-dichlor-phenylamino)-5-methyl-dihydro-furan-2-on,
3-(3,5-Dichlor-phenylamino)-5-(4-iod-phenyl)-5-methyl-dihydro-furan-2-on,
3-(3,5-Dichlor-phenylaminoF5-methyl-5-naphthalin-1-yl-dihydro-furan-2-on,
5-(3-Chlor-phenyl)-5-methyl-3-phenylamino-dihydro-furan-2-on,
3-(2-Brom-4-methyl-phenylamino)-5-(4-iod-phenyl)-5-methyl-dihydro-furan-2-on,
3-(2-Brom-4-methyl-phenylamino)-5-methyl-5-naphthalin-1-yl-dihydro-furan-2-on,
3-(5-Chlor-2-methyl-phenylamino)-5-methyl-5-(5,6,7,8-tetrahydro-naphthalin-2-yl)-dihydro-furan-2-on,
3-(4-Brom-2-fluor-phenylamino)-5-isopropyl-5-phenyl-dihydro-furan-2-on,
5-(2,5-Dimethoxy-phenyl)-5-methyl-3-(5-trifluormethyl-pyridin-2-ylamino)-dihydro-furan-2-on,
5-(3,5-Dimethoxy-phenyl)-5-methyl-3-(5-trifluormethyl-pyridin-2-ylamino)-dihydro-furan-2-on,
3-(3-Brom-5-methyl-pyridin-2-ylamino)-5-(2-methoxy-phenyl)-5-methyl-dihydrofuran-2-on,
3-(3-Brom-5-methyl-pyridin-2-ylamino)-5-(2,5-dimethoxy-phenyl)-5-methyldihydro-furan-2-on,
3-(3-Brom-5-methyl-pyridin-2-ylamino)-5-(3,5-dimethoxy-phenyl)-5-methyldihydro-furan-2-on,
3-(5-Brom-3-methyl-pyridin-2-ylamino)-5-(2-methoxy-phenyl)-5-methyl-dihydrofuran-2-on,
3-(2-Chlor-pyridin-3-ylamino)-5-(2-methoxy-phenyl)-5-methyl-dihydro-furan-2-on,
3-(5-Brom-pyridin-2-ylamino)-5-(2,5-dimethoxy-phenyl)-5-methyl-dihydro-furan-2-on,
3-(3-Chlor-5-trifluormethyl-pyridin-2-ylamino)-5-(2,5-dimethoxy-phenyl)-5-methyl-dihydro-furan-2-on,
5-(2-Methoxy-phenyl)-5-methyl-3-(pyridin-2-ylamino)-dihydro-furan-2-on,
3-[5-(3-Brom-phenyl)-5-methyl-2-oxo-tetrahydro-furan-3-ylamino]-5-methylsulfanyl-pyrazol-4-carbonitril,
3-[5-(2,5-Dimethoxy-phenyl)-5-methyl-2-oxo-tetrahydro-furan-3-ylamino]-pyrazol-4-carbonitril,
3-(4-Brom-pyrazol-3-ylamino)-5-(3,5-dimethoxy-phenyl)-5-methyl-dihydro-furan-2-on,
3-(4-Brom-5-phenyl-2H-pyrazol-3-ylamino)-5-(2-methoxy-phenyl)-5-methyldihydro-furan-2-on,
3-(8-Hydroxy-chinolin-2-ylamino)-5-(2-methoxy-phenyl)-5-methyl-dihydro-furan-2-on,
5-(2,5-Dimethoxy-phenyl)-3-(8-hydroxy-chinolin-2-ylamino)-5-methyl-dihydrofuran-2-on,
5-(2-Methoxy-phenyl)-5-methyl-3-(pyrazin-2-ylamino)-dihydro-furan-2-on,
5-(3-Brom-phenyl)-5-methyl-3-(4-methyl-pyrimidin-2-ylamino)-dihydro-furan-2-on,
5-(2-Methoxy-phenyl)-5-methyl-3-(pyrimidin-2-ylamino)-dihydro-furan-2-on,
3-(4-Chlor-3-trifluormethyl-phenylamino)-5-phenyl-5-propyl-dihydro-furan-2-on,
3-(2-Chlor-phenylamino)-5-phenyl-5-propyl-dihydro-furan-2-on,
3-(2-Chlor-4-fluor-phenylamino)-5-phenyl-5-propyl-dihydro-furan-2-on,
3-(4-Chlor-2-fluor-phenylamino)-5-phenyl-5-propyl-dihydro-furan-2-on,
3-(2-Chlor-4-methyl-phenylamino)-5-phenyl-5-propyl-dihydro-furan-2-on,
3-(4-Brom-5-phenyl-pyrazol-3-ylam ino)-5-phenyl-5-propyl-d ihydro-fu ra n-2-on,
5-Methylsulfanyl-3-(2-oxo-5-phenyl-5-propyl-tetrahydro-furan-3-ylamino)-pyrazol-4-carbonitril,
3-(4-Brom-5-phenyl-pyrazol-3-ylamino)-5-butyl-5-phenyl-dihydro-furan-2-on,
3-(5-Butyl-2-oxo-5-phenyl-tetrahydro-furan-3-ylamino)-5-methylsulfanyl-pyrazol-4-carbonitril,
3-(5-Butyl-2-oxo-5-phenyl-tetrahydro-furan-3-ylamino)-pyrazol-4-carbonitril,
5-Butyl-3-(2-phenoxy-phenylamino)-5-phenyl-dihydro-furan-2-on,
5-Biphenyl-4-yl-3-(2,4-dichlor-phenylamino)-5-methyl-dihydro-furan-2-on,
5-Biphenyl-4-yl-3-(2-chlor-phenylamino)-5-methyl-dihydro-furan-2-on,
5-Biphenyl-4-yl-3-(2-chlor-4-fluor-phenylamino)-5-methyl-dihydro-furan-2-on,
5-Biphenyl-4-yl-3-(4-brom-5-phenyl-pyrazol-3-ylamino)-5-methyl-dihydro-furan-2-on,
3-(3,5-Dichlorphenylamino)-5-methyl-5-phenyl-dihydrofuran-2-on,
3-(3,5-Dichlorphenylamino)-5-methyl-5-o-tolyl-dihydrofuran-2-on,
3-(3,5-Dichlorphenylamino)-5-(4-fluorphenyl)-5-methyl-dihydrofuran-2-on,
5-(2-Chlorphenyl)-3-(3,5-dichlorphenylamino)-5-methyl-dihydrofuran-2-on,
5-(4-Chlorphenyl)-3-(3,5-dichlorphenylamino)-5-methyl-dihydrofuran-2-on,
5-(3-Bromphenyl)-3-(3,5-dichlorphenylamino)-5-methyl-dihydrofuran-2-on,
5-(4-Bromphenyl)-3-(3,5-dichlorphenylamino)-5-methyl-dihydrofuran-2-on,
3-(3,5-Dichlorphenylamino)-5-(4-iodphenyl)-5-methyl-dihydrofuran-2-on,
3-(3,5-Dichlorphenylamino)-5-(2-methoxyphenyl)-5-methyf-dihydrofuran-2-on,
3-(3,5-Dichlorphenylamino)-5-(3-methoxyphenyl)-5-methyl-dihydrofuran-2-on,
3-(3,5-DichlorphenylaminoF5-(4-methoxyphenyl)-5-methyl-dihydrofuran-2-on,
3-(3,5-Dichlorphenylamino)-5-(2,4-dimethoxyphenyl)-5-methyl-dihydrofuran-2-on,
3-(3,5-Dichlorphenylamino)-5-(2,5-dimethoxyphenyl)-5-methyl-dihydrofuran-2-on,
3-(3,5-Dichlorphenylamino)-5-(3,5-dimethoxyphenyl)-5-methyl-dihydrofuran-2-on,
5-(Biphenyl-4-yl-)3-(3,5-dichlorphenylamino)-5-methyl-dihydrofuran-2-on,
3-(3,5-Dichlorphenylamino)-5-ethyl-5-phenyl-dihydrofuran-2-on,
3-(3,5-Dichlorphenylamino)-5-phenyl-5-n-propyl-dihydrofuran-2-on,
5-n-Butyl-3-(3,5-dichlorphenylamino)-5-phenyl-dihydrofuran-2-on,
3-(3,5-Dichlorphenylamino)-7a-phenyl-hexahydrobenzofuran,
3-(3,5-Dichlorphenylamino)-7a-(3-methoxy-phenyl)-hexahydrobenzofuran-2-on
   und
3-(3,5-Dichlorphenylamino)-8a-(3-methoxy-phenyl)-octahydrocyclohepta[b]furan-2-on
   sowie jeweils deren entsprechende physiologisch verträgliche Salze, insbesondere deren Hydrochloride.

Ein weiterer Gegenstand der vorliegenden Erfindung ist ein Verfahren zur Herstellung der erfindungsgemäßen γ-Lactonverbindungen der allgemeinen Formel I, worin die Reste R¹ bis R⁴ die oben angegebene Bedeutung haben, gemäß dem wenigstens eine Aminkomponente der allgemeinen Formel II 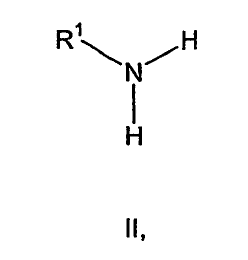 worin der Rest R¹ die oben angegebene Bedeutung hat, mit Glyoxalsäure, vorzugsweise in Form des Monohydrats oder einer wäßrigen Lösung, und wenigstens einer Alkenkomponente der allgemeinen Formel III 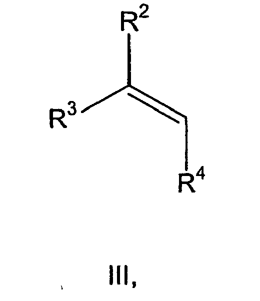 worin die Reste R² bis R⁴ die oben angegebene Bedeutung haben, in Gegenwart zumindest einer organischen und/oder anorganischen Säure, vorzugsweise Trifluoressigsäure in einem organischen Lösungsmittel zu wenigstens einer Verbindung der oben angegebenen allgemeinen Formel I umgesetzt und diese gegebenenfalls nach üblichen Methoden gereinigt und/oder gegebenenfalls nach üblichen Methoden isoliert wird.

Die Mengen der einzusetzenden Reaktionskomponenten der allgemeinen Formeln II und III, der Glyoxalsäure sowie der anorganischen und/oder organischen Säure, die Temperatur während der Umsetzung und die Dauer der Umsetzung können variieren. Die für die jeweilige Umsetzung geeignete Menge der einzusetzenden Komponenten, die geeignete Temperatur sowie die geeignete Dauer der Umsetzung können vom Fachman durch einfach Vorversuche ermittelt werden. Vorzugsweise beträgt die Temperatur während der Umsetzung 0 bis 100 °C, besonders bevorzugt 15 bis 40 °C. Die Dauer der Umsetzung beträgt vorzugsweise 0,25 bis 12 Stunden.

Als geeignetes Lösungsmittel wird bevorzugt Acetonitril oder ein Gemisch enthaltend Acetonitril eingesetzt.

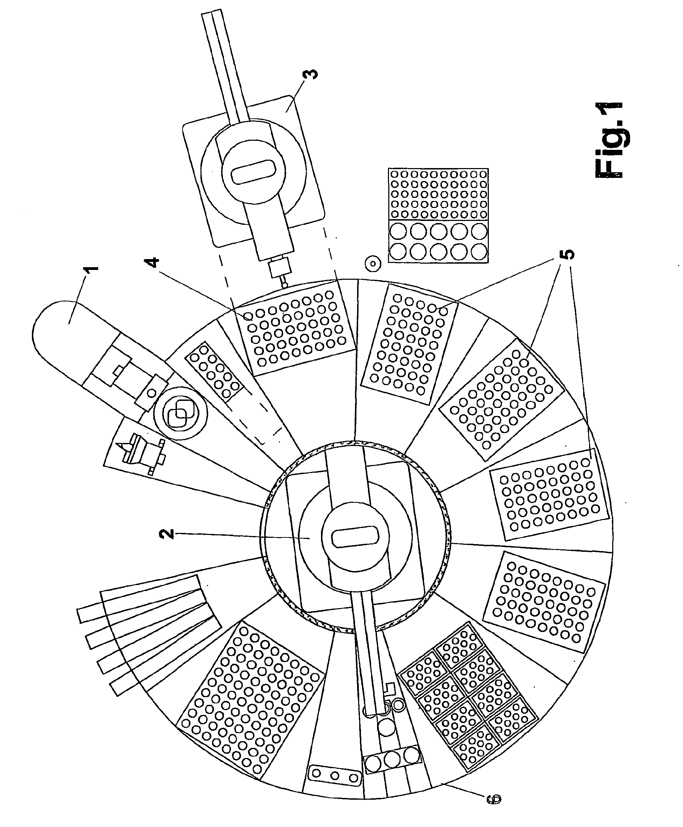

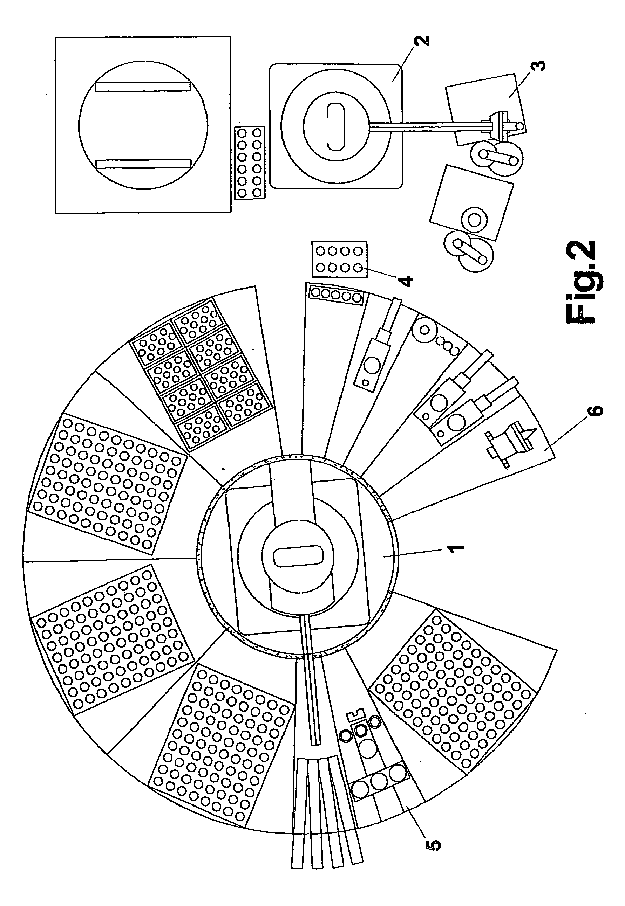

Vorzugsweise kann die Herstellung der erfindungsgemäßen substituierten γ-Lactonverbindungen auf einer automatischen Anlage der Firma Zymark gemäß **Figur 1** und **Figur 2** erfolgen, wie untenstehend beschrieben.

Anstelle der vorstehend beschriebenen Umsetzung der Reaktionskomponenten der allgemeinen Formeln II und III sowie Glyoxalsäure in Gegenwart zumindest einer organischen und/oder einer anorganischen Säure kann auch eine Umsetzung dieser Reaktionskomponenten, ggf. in Gegenwart zumindest einer anorganischen und/oder organischen Säure unter Bestrahlung mit Mikrowellen oder unter Einwirkung von Ultraschall erfolgen.

Ein weiterer Gegenstand der vorliegenden Erfindung ist daher ein Verfahren zur Herstellung der erfindungsgemäßen γ-Lactonverbindungen der allgemeinen Formel I, worin die Reste R¹ bis R⁴ die oben angegebene Bedeutung haben, gemäß dem wenigstens ein Aminkomponente der vorstehend angegebenen allgemeinen Formel II, worin R¹ die oben angegebene Bedeutung hat, mit Glyoxalsäure, vorzugsweise in Form des Monohydrats oder einer wäßrigen Lösung, und wenigstens einer Alkenkomponente der oben angegebenen allgemeinen Formel III, worin R² bis R⁴ die oben angegebene Bedeutung haben, in einem organischen Lösungsmittel, ggf. in Gegenwart zumindest einer anorganischen und/oder organischen Säure unter Mikrowellenbestrahlung oder unter Einwirkung von Ultraschall, vorzugsweise unter Mikrowellenbestrahlung, zu wenigstens einer Verbindung der oben angegebenen allgemeinen Formel I umgesetzt und diese gegebenenfalls nach üblichen Methoden gereinigt und/oder gegebenenfalls nach üblichen Methoden isoliert wird.

Die Mengen der einzusetzenden Reaktionskomponenten der allgemeinen Formeln II und III sowie der Glyoxalsäure, die geeignete Temperatur während der Umsetzung und die geeignete Dauer der Umsetzung können variieren. Die für die jeweilige Umsetzung optimale Menge der einzusetzenden Komponenten, die optimale Temperatur sowie die optimale Dauer der Umsetzung können vom Fachman durch einfach Vorversuche ermittelt werden. Sofern die Umsetzung unter Mikrowellenbestrahlung erfolgt, beträgt die Temperatur vorzugsweise 40 bis 70 °C, besonders bevorzugt 45 bis 60 °C. Die Dauer der Umsetzung beträgt vorzugsweise 0,1 bis 60 Minuten für die Bestrahlung mit Mikrowellen.

Als geeignetes Lösungsmittel wird bevorzugt Acetonitril oder ein Gemisch enthaltend Acetonitril eingesetzt.

Die jeweiligen Reaktionskomponenten der allgemeinen Formeln II und III sowie Glyoxalsäure können käuflich am Markt erworben oder nach üblichen, dem Fachmann bekannten Methoden hergestellt werden.

Die erfindungsgemäßen substituierten γ-Lactonverbindungen der allgemeinen Formel I können nach dem erfindungsgemäßen Verfahren als freie Base oder als Salz isoliert werden. Die freie Base der jeweiligen erfindungsgemäßen Verbindung der allgemeinen Formel I kann nach üblichen, dem Fachmann bekannten Methoden, beispielsweise durch Umsetzung mit einer anorganischen oder organischen Säure, vorzugsweise mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Milchsäure, Citronensäure, Glutaminsäure oder Asparaginsäure, in das entsprechende, physiologisch verträgliche Salz übergeführt werden. Ferner kann die freie Base auch durch Umsetzung mit

Die freie Base der jeweiligen erfindungsgemäßen Verbindung der allgemeinen Formel I kann auch mit der freien Säure oder einem Salz eines Zuckerersatzstoffes, wie z.B. Saccharin, Cyclamat oder Acesulfam, in das entsprechende physiologisch verträgliche Salz übergeführt werden.

Die Überführung der freien Base der jeweiligen erfindungsgemäßen Verbindung der allgemeinen Formel I in das entsprechende Hydrochlorid kann bevorzugt auch durch Versetzen der in einem geeigneten organischen Lösungsmittel, wie z.B. Butan-2-on (Methylethylketon), gelösten erfindungsgemäßen Verbindung der allgemeinen Formel I als freie Base mit Trimethylsilylchlorid (TMSCI) erhalten werden.

Sofern die erfindungsgemäßen substituierten γ-Lactonverbindungen der allgemeinen Formel I nach dem erfindungsgemäßen Herstellungsverfahren in Form ihrer Racemate oder anderer Mischungen ihrer verschiedenen Enantiomeren und/oder Diastereomeren erhalten werden, können diese, sofern erforderlich, nach üblichen, dem Fachmann bekannten Verfahren getrennt und ggf. isoliert werden. Beispielhaft seien chromatographische Trennverfahren, insbesondere Flüssigkeitschromatographie-Verfahren unter Normaldruck oder unter erhöhtem Druck, bevorzugt MPLC- und HPLC-Verfahren, sowie Verfahren der fraktionierten Kristallisation genannt. Dabei können insbesondere einzelne Enantiomeren, z.B. mittels HPLC an chiraler Phase oder mittels Kristallisation mit chiralen Säuren, etwa (+)-Weinsäure, (-)-Weinsäure oder (+)-10-Camphersulfonsäure, gebildete diastereomere Salze voneinander getrennt werden.

Die erfindungsgemäßen γ-Lactonverbindungen der allgemeinen Formel I sind toxikologisch unbedenklich und eignen sich daher als pharmazeutische Wirkstoffe in Arzneimitteln.

Ein weiterer Gegenstand der vorliegenden Erfindung sind daher Arzneimittel, die wenigstens eine erfindungsgemäße γ-Lactonverbindung der allgemeinen Formel I einschließlich der vorstehend ausgenommenen Verbindungen sowie gegebenenfalls physiologisch verträgliche Hilfsstoffe enthalten.

Vorzugsweise eignen sich die erfindungsgemäßen Arzneimittel zur Bekämpfung von Schmerz, besonders bevorzugt zur Bekämpfung von chronischem oder neuropathischem Schmerz.

Ebenfalls bevorzugt eignen sich die erfindungsgemäßen Arzneimittel zur Behandlung oder Prophylaxe von neurodegenerativen Erkrankungen, insbesondere von Morbus Alzheimer, Morbus Parkinson oder Morbus Huntington oder zur Behandlung oder Prophylaxe von Migräne, Schlaganfall, cerebraler Ischämie, cerebralem Infarkt, Hirnödem, Schizophrenie, Psychosen bedingt durch erhöhten Aminosäurespiegel, AIDS-Demenz, Tourette-Syndrom, inflammatorischen und/oder allergischen Reaktionen, Depressionen, seelischen Erkrankungen, Epilepsie, Harninkontinenz, Juckreiz, Tinnitus aurium, Diarrhoe, zur Anxiolyse oder zur Anästhesie.

Die Verwendung wenigstens einer substituierten γ-Lactonverbindung der allgemeinen Formel I einschließlich der vorstehend ausgenommenen Verbindungen zur Herstellung eines Arzneimittels zur Bekämpfung von Schmerz, insbesondere zur Bekämpfung von chronischem oder neuropathischem Schmerz, zur Behandlung oder Prophylaxe von neurodegenerativen Erkrankungen, insbesondere von Morbus Alzheimer, Morbus Parkinson oder Morbus Huntington oder zur Behandlung oder Prophylaxe von Migräne, Schlaganfall, cerebraler Ischämie, cerebralem Infarkt, Hirnödem, Schizophrenie, Psychosen bedingt durch erhöhten Aminosäurespiegel, AIDS-Demenz, Tourette-Syndrom, inflammatorischen und/oder allergischen Reaktionen, Depressionen, seelischen Erkrankungen, Epilepsie, Haminkontinenz, Juckreiz, Tinnitus aurium, Diarrhoe, zur Anxiolyse oder Anästhesie ist daher ebenfalls Gegenstand der vorliegenden Erfindung.

Die erfindungsgemäßen Arzneimittel können auch Mischungen verschiedener Stereoisomere einer oder mehrerer erfindungsgemäßer γ-Lactonverbindungen enthalten. So können beispielsweise auch verschiedene Enantiomeren einer erfindungsgemäßen γ-Lactonverbindung in nicht äquimolaren Mengen vorliegen.

Die erfindungsgemäßen Arzneimittel enthalten neben mindestens einer erfindungsgemäßen substituierten γ-Lactonverbindung üblicherweise weitere physiologisch verträgliche Hilfsstoffe, die bevorzugt ausgewählt sind aus der Gruppe bestehend aus Trägermaterialien, Füllstoffen, Lösungsmitteln, Verdünnungsmitteln, Farbstoffen und Bindemittein. Die erfindungsgemäßen Arzneimittel können als flüssige, halbfeste oder feste Arzneiformen, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, vorliegen und als solche auch verabreicht werden.

Die Auswahl der physiologisch verträglichen Hilfsstoffe sowie die einzusetzenden Mengen derselben hängt davon ab, ob das Arzneimittel oral, peroral, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße substituierte γ-Lactonderivate in einem Depot in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen substituierten γ-Lactonderivate verzögert freisetzen.

Die Herstellung der erfindungsgemäßen Arzneimittel erfolgt mit Hilfe von üblichen, dem Fachmann bekannten Mitteln, Vorrichtungen, Methoden und Verfahren, wie sie beispielsweise in "Remington's Pharmaceutical Sciences", Hrsg. A.R. Gennaro, 17. Ed., Mack Publishing Company, Easton, Pa. (1985), insbesondere in Teil 8, Kapitel 76 bis 93, beschrieben sind. Die entsprechende Literaturbeschreibung wird hiermit als Referenz eingeführt und gilt als Teil der Offenbarung.

Die an den Patienten zu verabreichende Menge der jeweiligen erfindungsgemäßen Verbindung der allgemeinen Formel I kann variieren, beispielsweise in Abhängigkeit vom Gewicht oder dem Alter des Patienten sowie von der Applikationsart, der Indikation und dem Schweregrad der Erkrankung. Üblicherweise werden 0,5 bis 500 mg pro kg Körpergewicht des Patienten wenigstens einer erfindungsgemäßen Verbindung der allgemeinen Formel I appliziert..

### Molekularpharmakologische Untersuchungen:

Die Untersuchungen zur Bestimmung der Affinität der erfindungsgemäßen γ-Lactonverbindungen der allgemeinen Formel I zur Glycin-Bindungsstelle des NMDA-Rezeptorkanals wurde an Hirnmembran-homogenaten (Homogenat von Cortex- und Hippocampus-Areal aus dem Hirn von männlichen Ratten, Stamm Wistar, Charles River, WIGA GmbH, Sulzbach, Deutschland) durchgeführt, wie in B.M. Baron et al., Journal of Pharmacology and Experimental Therapeutics, Vol. 279, Seiten 62-68, 1996 beschrieben. Die entsprechende Literaturbeschreibung wird hiermit als Referenz eingeführt und gilt als Teil der Offenbarung.

Hierzu wurde Cortex und Hippocampus aus frisch entnommenen Rattengehimen freipräpariert und in 5 mmol/l TRIS-Acetatpuffer, 0,32 mol/l Sacharose pH 7,4 (10 ml/g Frischgewicht) mit einem Potter-Homogenisator (Firma Braun, Melsungen, Deutschland, 10 Kolbenhübe bei 500 Umdrehungen pro Minute (Upm)) unter Eiskühlung homogenisiert und anschließend für 10 Minuten bei 1.000 g und 4 °C zentrifugiert. Der erste Überstand wurde gesammelt und das Sediment erneut mit 5 mmol/l TRIS-Acetatpuffer, 0,32 mol/l Sacharose pH 7,4 (5 ml/g ursprüngliche Frischeinwaage Rattenhirn-Cortex und Hippocampus) mit dem Potter-Homogenisator (10 Kolbenhübe bei 500 Upm) unter Eiskühlung homogenisiert und für 10 Minuten bei 1.000 g und 4°C zentrifugiert. Der resultierende Überstand wurde mit dem Überstand aus der ersten Zentrifugation vereinigt und bei 17.000 g für 20 Minuten bei 4°C zentrifugiert. Der Überstand nach dieser Zentrifugation wurde verworfen und das Membransediment mit 5 mmol/l TRIS-Acetatpuffer pH 8,0 (20 ml/g ursprüngliches Frischgewicht) aufgenommen und mit 10 Kolbenhüben bei 500 Upm homogenisiert.

Anschließend wurde das Membranhomogenat für 1 Stunde bei 4 °C inkubiert für 30 Minuten bei 50.000 g und 4 °C zentrifugiert. Der Überstand wurde verworfen und das Zentrifugenröhrchen mit dem Membransediment mit Parafilm verschlossen und für 24 Stunden bei -20 °C eingefroren. Am folgenden Tag wurde das Membransediment aufgetaut und mit eiskaltem 5 mmol/l TRIS-Acetatpuffer, 0,1 % Saponin (Gewicht/Volumen) pH 7,0 (10 ml/g ursprüngliche Frischeinwaage) aufgenommen und mit 10 Kolbenhüben bei 500 Upm homogenisiert und anschließend für 20 Minuten bei 50.000 g und 4°C zentrifugiert. Der resultierende Überstand wurde verworfen und das Sediment in einem kleinen Volumen mit 5 mmol/l TRIS-Acetatpuffer pH 7,0 (ca. 2 ml/g ursprüngliches Frischgewicht) aufgenommen und erneut mit 10 Kolbenhüben bei 500 Upm homogenisiert. Nach Bestimmung des Proteingehaltes wurde das Membranhomogenat mit 5 mmol/l TRIS-Acetatpuffer pH 7,0 auf eine Proteinkonzentration von 10 mg Protein/ml eingestellt und in Aliquoten bis zur Testung eingefroren.

Für den Rezeptorbindungstest wurden Aliquote aufgetaut, 1:10 mit 5 mmol/l TRIS-Acetatpuffer pH 7,0 verdünnt, mit 10 Kolbenhüben bei 500 Upm mit dem Potter-Homogenisator unter Eiskühlung homogenisiert und für 60 Minuten bei 55.000 g bei 4°C zentrifugiert. Der Überstand wurde dekantiert und das Membransediment mit eiskaltem 50 mmol/l TRIS-Acetatpuffer pH 7,0 auf eine Proteinkonzentration von 1 mg/ml eingestellt und erneut mit 10 Kolbenhüben bei 500 Upm homogenisiert und unter Rühren auf einem Magnetrührer im Eisbad in Suspension gehalten. Von diesem Membranhomogenat wurden jeweils 100 µl je 1 ml-Ansatz im Rezeptorbindungstest eingesetzt (0,1 mg Protein/ml im Endansatz).

Im Bindungstest wurde als Puffer 50 mmol/l TRIS-Acetatpuffer pH 7,0 sowie als radioaktiver Ligand 1 nmol/l (³H)-MDL 105.519, wie in der vorstehend angegebenen Literatur von B.M. Baron et al. beschrieben, eingesetzt. Der Anteil an unspezifischer Bindung wurde in Anwesenheit von 1 mmol/l Glycin bestimmt.

In weiteren Ansätzen wurden die erfindungsgemäßen Verbindungen der allgemeinen Formel I in Konzentrationsreihen zugegeben und die Verdrängung des radioaktiven Liganden aus seiner spezifischen Bindung an die Glycin-Bindungsstelle des NMDA-Rezeptorkanals ermittelt. Die jeweiligen Dreifachansätze wurden über 120 Minuten bei 4°C inkubiert und anschließend zur Bestimmung des an das Membranhomogenat gebundenen radioaktiven Liganden mittels Filtration durch Glasfaser-Filtermatten (Typ Whatman GF/B, Fa. Adi Hassel, München, Deutschland) geemtet. Die auf den Glasfaser-Filtern zurückgehaltene Radioaktivität wurde nach Zugabe von Szintillator (Ready Protein, Fa. Beckamnn Coulter GmbH, Krefeld, Deutschland) im β-Counter (Packard TRI-CARB Liquid Szintillation Analyzer 2000CA, Fa. Packard Instrument, Meriden, CT 06450, USA) gemessen.

Die Affinität der erfindungsgemäßen Verbindungen der allgemeinen Formel I zur Glycin-Bindungsstelle des NMDA-Rezeptorkanals wurde als IC₅₀ (Konzentration mit 50 % Verdrängung des radioaktiven Liganden aus seiner spezifischen Bindung) nach dem Massenwirkungsgesetz mittels nichtlinearer Regression berechnet und wird nach Umrechnung gemäß der Cheng-Prussoff-Beziehung, beschrieben in Y. Cheng, W.H. Prusoff, 1973, Biochem. Pharmacol., Vol. 22, Seiten 3099-3108, als Ki-Wert angegeben.

Im folgenden wird die vorliegende Erfindung anhand von Beispielen erläutert. Diese Erläuterungen sind lediglich beispielhaft und schränken den allgemeinen Erfindungsgedanken nicht ein.

### Beispiele:

Die zur Herstellung der erfindungsgemäßen γ-Lactonverbindungen eingesetzten Chemikalien und Lösungsmittel wurden kommerziell, beispielsweise von Acros, Avocado, Aldrich, Fluka, Lancaster, Maybridge, Merck, Sigma oder TCI, erworben oder nach üblichen, dem Fachmann bekannten Methoden hergestellt.

Die dünschichtchromatographischen Untersuchungen wurden mit HPTLC-Fertigplatten, Kieselgel 60 F 254, der Firma E. Merck, Darmstadt, durchgeführt.

Die Ausbeuten der hergestellten Verbindungen wurden nicht optimiert.

Die Analytik erfolgte über ESI-Massenspektroskopie oder NMR-Spektroskopie.

### Allgemeine Arbeitsvorschrift 1:

Die Synthese der erfindungsgemäßen γ-Lactonverbindungen erfolgte auf einer automatischen Anlage der Firma Zymark gemäß **Figur 1** und **Figur 2.**

Dabei umfaßt Figur 1 eine Capper-Station (Ziff. 1) zum Verschließen der Reaktionsröhrchen, einen Roboter 1 (Ziff. 2) und einen Roboter 2 (Ziff. 3), wobei der Roboter 1 die Reaktionsröhrchen bzw. die entsprechenden Racks bewegt und der Roboter 2 die Reagenzien in die Reaktionsröhrchen pipettiert, einen temperierbaren Reaktorblock (Ziff. 4), Rührblöcke (Ziff. 5) und eine Filtrationsstation (Ziff. 6), in der die Reaktionslösung abfiltriert wird.

Figur 2 umfaßt ebenfalls einen Roboter 1 (Ziff. 1) und einen Roboter 2 (Ziff. 2), die beide die Glasröhrchen mit den Syntheseprodukten auf die verschiedenen Stationen bewegen. Bei den Stationen handelt es sich im einzelnen um einen Vortexer (Ziff. 3) zum Durchmischen der Proben und zum Zudosieren von Lösungen oder Lösungsmitteln, einen Spin-Reaktor (Ziff. 4) zur Durchmischung von Proben, eine Phasendetektionsstation (Ziff. 5) zur Detektion der Phasengrenze und Phasentrennung sowie eine Station (Ziff. 6) zum Trocknen der Syntheseprodukte über Salzkartuschen.

Zur Synthese wurde ein Rundbodenröhrchen aus Glas (Durchmesser 16 mm, Länge 125 mm) mit Gewinde manuell mit einem Rührer versehen und auf der Capper-Station (Ziff. 1) gemäß Figur 1 mit einem Schraubdeckel mit Septum verschlossen. Das Röhrchen wurde von Roboter 1 (Ziff. 2) in den auf 20 °C temperierten Reaktorblock (Ziff. 4) gestellt. Roboter 2 (Ziff. 3) pipettierte nacheinander folgende Reagenzien hinzu:
1.) 1 ml einer Lösung aus Trifluoressigsäure und der jeweiligen Aminkomponente, jeweils 0,1 M in Acetonitril,
2.) 1 ml einer 0,11 M Glyoxalsäure-Monohydrat-Lösung in Acetonitril
3.) 1 ml einer 0,3 M Lösung der jeweiligen Alkenkomponente in Acetonitril

Das Reaktionsgemisch wurde im Anschluss bei 20 °C in einem der Rührblöcke (Ziff. 5) 600 min lang gerührt. Danach wurde die Reaktionslösung an der Filtrations-Station (Ziff. 6) abfiltriert. Das Röhrchen wurde dabei zweimal mit je 1,5 ml einer 7,5 Gew.-% Natriumhydrogencarbonat-Lösung gespült.

Das Rack mit den Röhrchen wurde anschließend manuell auf eine automatische Aufarbeitungsanlage gemäß Figur 2 gestellt. Dort wurde das Reaktionsgemisch auf einem Vortexer (Ziff. 3) mit 2 ml Diethylether versetzt und geschüttelt.

Im Spin-Reaktor (Ziff. 4) wurde zehn Minuten lang gründlich gemischt und durch die langsame Abnahme der Drehbewegung eine deutliche-Phasengrenze ausgebildet. Diese Phasengrenze wurde auf der Phasendetektionsstation (Ziff. 5) optisch detektiert und die organische Phase abpipettiert. Im nächsten Schritt wurde die wäßrige Phase erneut mit 2 ml Diethylether versetzt, geschüttelt, zentrifugiert und die organische Phase abpipettiert. Die vereinigten organischen Phasen wurden über 2,4 g MgSO₄ (granuliert) getrocknet. Das Lösungsmittel wurde in einer Vakuumzentrifuge entfernt. Jede Probe wurde anschließend mit Elektronenspray-Ionisations-Massenspektrometrie (ESI-MS) und/oder NMR-Spektroskopie analysiert.

Durch die automatisierte Synthese ist eine Gleichbehandlung aller Proben sowie eine konstante Reaktionsführung gewährleistet. Die nach der vorstehenden allgemeinen Arbeitsvorschrift hergestellten beispielgemäßen γ-Lactonverbindungen sind in der nachfolgenden Tabelle 1 angegeben:

**Tabelle 1:**

| **Beispiel** | **Name** | **berechnete Masse** | **gefundene Masse (ESI)** |
|---|---|---|---|
| 1 | 3-(2-Chlor-4-fluor-phenylamino)-5-(4-fluor-phenyl)-5-methyl-dihydro-furan-2-on | 337.75 | 338,1 |
| 2 | 5-Methyl-3-(4-phenoxy-phenylamino)-5-phenyl-dihydro-furan-2-on | 359.42 | 360,1 |
| 3 | 3-(2-Chlor-phenylamino)-5-(4-fluor-phenyl)-5-methyl-dihydro-furan-2-on | 319.76 | 320,2 |
| 4 | 3-(4-Chlor-2-methyl-phenylamino)-5-methyl-5-phenyl-dihydro-furan-2-on | 315.8 | 316,1 |
| 5 | 3-(2,4-Dichlor-phenylamino)-5-(4-fluor-phenyl)-5-methyl-dihydro-furan-2-on | 354.21 | 354,2/ 356,1 |
| 6 | 3-(4-Chlor-3-trifluormethyl-phenylamino)-5-methyl-5-phenyl-dihydro-furan-2-on | 369.77 | 370,1/ 372,1 |
| 7 | 3-(2,3-Dichlor-phenylamino)-5-(4-fluor-phenyl)-5-methyl-dihydro-furan-2-on | 354.21 | 354,1/ 356,1 |
| 8 | 3-(4-lod-phenylamino)-5-methyl-5-phenyl-dihydro-furan-2-on | 393.22 | 394,0 |
| 9 | 3-(4-Chlor-2-fluor-phenylamino)-5-(4-fluor-phenyl)-5-methyl-dihydro-furan-2-on | 337.75 | 338,1 |
| 10 | 3-(2-Chlor-4-methyl-phenylamino)-5-methyl-5-phenyl-dihydro-furan-2-on | 315.8 | 316,2 |
| 11 | 3-(2-Chlor-4-methyl-phenylamino)-5-(4-fluor-phenyl)-5-methyl-dihydro-furan-2-on | 333.79 | 334,2 |
| 12 | 3-(3,5-Dichlor-phenylamino)-5-methyl-5-phenyl-dihydro-furan-2-on | 336.22 | 336,1/ 338,0 |
| 13 | 3-(3,5-Dichlor-phenylamino)-5-(4-fluor-phenyl)-5-methyl-dihydro-furan-2-on | 354.21 | 354,0 |
| 14 | 3-(4-Brom-2-chlor-phenylamino)-5-(4-fluor-phenyl)-5-methyl-dihydro-furan-2-on | 398.66 | 398,1/ 400,0 |
| 15 | 4-(5-Methyl-2-oxo-5-phenyl-tetrahydro-furan-3-ylamino)-benzonitril | 292.34 | 293,3 |
| 16 | 5-(4-Chlor-phenyl)-3-(4-iod-phenylamino)-5-methyl-dihydro-furan-2-on | 427.67 | 428,0 |
| 17 | 5-(4-Chlor-phenyl)-3-(2,4-dichlor-phenylamino)-5-methyl-dihydro-furan-2-on | 370.66 | 370,1/ 372,1 |
| 18 | 5-(4-Chlor-phenyl)-3-(2-chlor-phenylamino)-5-methyl-dihydro-furan-2-on | 336.22 | 336,2/ 338,0 |
| 19 | 3-(4-Chlor-2-methyl-phenylamino)-5-(4-chlor-phenyl)-5-methyl-dihydro-furan-2-on | 350.24 | 350,2/ 352,0 |
| 20 | 3-(2-Chlor-4-fluor-phenylamino)-5-(4-chlor-phenyl)-5-methyl-dihydro-furan-2-on | 354.21 | 354,1/ 356,1 |
| 21 | 3-(4-Chlor-2-fluor-phenylamino)-5-(4-chlor-phenyl)-5-methyl-dihydro-furan-2-on | 354.21 | 354,1/ 356,1 |
| 22 | 3-(2-Chlor 4-methyl-phenylamino)-5-(4-chlor-phenyl)-5-methyl-dihydro-furan-2-on | 350.24 | 350,1/ 352,0 |
| 23 | 5-(4-Chlor-phenyl)-3-(2,3-dichlor-phenylamino)-5-methyl-dihydro-furan-2-on | 370.66 | 370,1/ 372,1 |
| 24 | 3-(4-Brom-2-chlor-phenylamino)-5-(4-chlor-phenyl)-5-methyl-dihydro-furan-2-on | 415.12 | 414,1/416, 0/418,0 |
| 25 | 5-(4-Chlor-phenyl)-3-(3,5-dichlor-phenylamino)-5-methyl-dihydro-furan-2-on | 370.66 | 370,1/ 372,0 |
| 26 | 3-(3,5-Dibrom-pyridin-2-ylamino)-5-methyl-5-phenyl-dihydro-furan-2-on | 426.12. | 427,1 |
| 27 | 5-(4-Chlor-phenyl)-3-(3,5-dichlor-pyridin-2-ylamino)-5-methyl-dihydro-furan-2-on | 371.65 | 371,0/ 373,0 |
| 28 | 5-(4-Chlor-phenyl)-5-methyl-3-(5-nitro-pyridin-2-ylamino)-dihydro-furan-2-on | 347.76 | 348,1 |
| 29 | 3-(3-Chlor-2-methyl-phenylamino)-5-(4-iod-phenyl)-5-methyl-dihydro-furan-2-on | 441.69 | 442,0 |
| 30 | 5-(4-Brom-phenyl)-3-(4-chlor-phenylamino)-5-methyl-dihydro-furan-2-on | 380.67 | 380,1/ 382,1 |
| 31 | 5-(3-Chlor-phenyl)-3-(4-chlor-phenylamino)-5-methyl-dihydro-furan-2-on | 336.21 | 336,1/ 338,1 |
| 32 | 3-(4-Chlor-phenylamino)-5-(4-iod-phenyl)-5-methyl-dihydro-furan-2-on | 427.66 | 428,0 |
| 33 | 5-(4-Brom-phenyl)-3-(2-iod-phenylamino)-5-methyl-dihydro-furan-2-on | 472.12 | 472,0/ 474,0 |
| 34 | 5-(3-Chlor-phenyl)-3-(2-iod-phenylamino)-5-methyl-dihydro-furan-2-on | 427.66 | 428,1 |
| 35 | 5-(4-Iod-phenyl)-3-(2-iod-phenylamino)-5-methyl-dihydro-furan-2-on | 519.11 | 519,9 |
| 36 | 3-(2,4-Difluor-phenylamino)-5-methyl-5-naphthalin-1-yl-dihydro-furan-2-on | 353.36 | 354,0 |
| 37 | 5-(4-Brom-phenyl)-3-(4-iod-phenylamino)-5-methyl-dihydro-furan-2-on | 472.12 | 472,0 |
| 38 | 5-(3-Chlor-phenyl)-3-(4-iod-phenylamino)-5-methyl-dihydro-furan-2-on | 427.66 | 428,0 |
| 39 | 3-(4-lod-phenylamino)-5-methyl-5-naphthalin-1-yl-dihydro-furan-2-on | 443.28 | 444,0 |
| 40 | 5-(4-Brom-phenyl)-3-(3,5-dichlor-phenylamino)-5-methyl-dihydro-furan-2-on | 415.11 | 414,0/ 416,0 |
| 41 | 5-(3-Chlor-phenyl)-3-(3,5-dichlor-phenylamino)-5-methyl-dihydro-furan-2-on | 370.66 | 370,0/ 372,0 |
| 42 | 3-(3,5-Dichlor-phenylamino)-5-(4-iod-phenyl)-5-methyl-dihydro-furan-2-on | 462.11 | 461,9/ 463,9 |
| 43 | 3-(3,5-Dichlor-phenylaminor5-methyl-5-naphthalin-1-yl-dihydro-furan-2-on | 386.27 | 386,0/ 387,9 |
| 44 | 5-(3-Chlor-phenyl)-5-methyl-3-phenylamino-dihydro-furan-2-on | 301.77 | 302,1 |
| 45 | 3-(2-Brom-4-methyl-phenylamino)-5-(4-iod-phenyl)-5-methyl-dihydro-furan-2-on | 486.15 | 485,9/ 487,9 |
| 46 | 3-(2-Brom-4-methyl-phenylamino)-5-methyl-5-naphthalin-1-yl-dihydro-furan-2-on | 410.31 | 410,0/ 412,0 |
| 47 | 3-(5-Chlor-2-methyl-phenylamino)-5-methyl-5-(5,6,7,8-tetrahydro-naphthalin-2-yl)-dihydrofuran-2-on | 369.89 | 370,1 |
| 48 | 3-(4-Brom-2-fluor-phenylamino)-5-isopropyl-5-phenyl-dihydro-furan-2-on | 392.27 | 392,1/ 394,1 |
| 49 | 5-(2,5-Dimethoxy-phenyl)-5-methyl-3-(5-trifluormethyl-pyridin-2-ylamino)-dihydro-furan2-on | 396.36 | 397,2 |
| 50 | 5-(3,5-Dimethoxy-phenyl)-5-methyl-3-(5-trifluormethyl-pyridin-2-ylamino)-dihydro-furan-2-on | 396.36 | 397,1 |
| 51 | 3-(3-Brom-5-methyl-pyridin-2-ylamino)-5-(2-methoxy-phenyl)-5-methyl-dihydro-furan-2-on | 391.26 | 391,1/ 393,1 |
| 52 | 3-(3-Brom-5-methyl-pyridin-2-ylamino)-5-(2,5-dimethoxy-phenyl)-5-methyl-dihydro-furan-2-on | 421.29 | 421,1 |
| 53 | 3-(3-Brom-5-methyl-pyridin-2-ylamino)-5-(3,5-dimethoxy-phenyl)-5-methyl-dihydro-furan-2-on | 421.29 | 421,1 |
| 54 | 3-(5-Brom-3-methyl-pyridin-2-ylamino)-5-(2-methoxy-phenyl)-5-methyl-dihydro-furan-2-on | 391.26 | 391,2/ 393,1 |
| 55 | 3-(2-Chlor-pyridin-3-ylamino)-5-(2-methoxy-phenyl)-5-methyl-dihydro-furan-2-on | 332.78 | 333,3 |
| 56 | 3-(5-Brom-pyridin-2-ylamino)-5-(2,5-dimethoxy-phenyl)-5-methyl-dihydro-furan-2-on | 407.26 | 407,1/ 409,1 |
| 57 | 3-(3-Chlor-5-trifluormethyl-pyridin-2-ylamino)-5-(2,5-dimethoxy-phenyl)-5-methyl-dihydro-furan-2-on | 430.8 | 431,1 |
| 58 | 5-(2-Methoxy-phenyl)-5-methyl-3-(pyridin-2-ylamino)-dihydro-furan-2-on | 298.34 | 299,2 |
| 61 | 3-[5-(3-Brom-phenyl)-5-methyl-2-oxo-tetrahydro-furan-3-ylamino]-5-methylsulfanyl-pyrazol-4-carbonitril | 407.29 | 407,1/ 409,0 |
| 62 | 3-[5-(2,5-Dimethoxy-phenyl)-5-methyl-2-oxo-tetrahydro-furan-3-ylamino]-pyrazol-4-carbonitril | 342.35 | 343,1 |
| 63 | 3-(4-Brom-pyrazo)-3-y)amino)-5-(3,5-dimethoxy-phenyl)-5-methyl-dihydro-furan-2-on | 396.24 | 396,1/ 398,1 |
| 64 | 3-(4-Brom-5-phenyl-2H-pyrazol-3-ylamino)-5-(2-methoxy-phenyl)-5-methyl-dihydro-furan-2-on | 442.31 | 442,1/ 444,1 |
| 65 | 3-(8-Hydroxy-chinolin-2-ylamino)-5-(2-methoxy-phenyl)-5-methyl-dihydro-furan-2-on | 364.4 | 365,2 |
| 66 | 5-(2,5-Dimethoxy-phenyl)-3-(8-hydroxy-chinolin-2-ylamino)-5-methyl-dihydro-furan-2-on | 394.42 | 395,2 |
| 67 | 5-(2-Methoxy-phenyl)-5-methyl-3-(pyrazin-2-ylamino)-dihydro-furan-2-on | 299.32 | 300,2 |
| 68 | 5-(3-Brom-phenyl)-5-methyl-3-(4-methyl-pyrimidin-2-ylamino)-dihydro-furan-2-on | 362.23 | 362,1/ 364,1 |
| 70 | 5-(2-Methoxy-phenyl)-5-methyl-3-(pyrimidin-2-ylamino)-dihydro-furan-2-on | 299.32 | 300,2 |
| 71 | 3-(4-Chlor-3-trifluormethyl-phenylamino)-5-phenyl-5-propyl-dihydro-furan-2-on | 397.82 | 398,0 |
| 72 | 3-(2-Chlor-phenylamino)-5-phenyl-5-propyl-dihydro-furan-2-on | 329.82 | 330,1 |
| 73 | 3-(2-Chlor-4-fluor-phenylamino)-5-phenyl-5-propyl-dihydro-furan-2-on | 347.81 | 348,1 |
| 74 | 3-(4-Chlor-2-fluor-phenylamino)-5-phenyl-5-propyl-dihydro-furan-2-on | 347.81 | 348,1 |
| 75 | 3-(2-Chlor-4-methyl-phenylamino)-5-phenyl-5-propyl-dihydro-furan-2-on | 343.85 | 344,1 |
| 78 | 3-(4-Brom-5-phenyl-pyrazol-3-ylamino)-5-phenyl-5-propyl-dihydro-furan-2-on | 440.34 | 440,1/ 442,1 |
| 79 | 5-Methylsulfanyl-3-(2-oxo-5-phenyl-5-propyl-tetrahydro-furan-3-ylamino)-pyrazol-4-carbonitril | 356.44 | 357,1 |
| 82 | 3-(4-Brom-5-phenyl-pyrazol-3-ylamino)-5-butyl-5-phenyl-dihydro-furan-2-on | 454.37 | 454,1 |
| 83 | 3-(5-Butyl-2-oxo-5-phenyl-tetrahydro-furan-3-ylamino)-5-methylsulfanyl-pyrazol-4-carbonitril | 370.47 | 371,1 |
| 84 | 3-(5-Butyl-2-oxo-5-phenyl-tetrahydro-furan-3-ylamino)-pyrazol-4-carbonitrile | 324.38 | 325,1 |
| 85 | 5-Butyl-3-(2-phenoxy-phenylamino)-5-phenyl-dihydro-furan-2-on | 401.5 | 402,1 |
| 86 | 5-Biphenyl-4-yl-3-(2,4-dichlor-phenylamino)-5-methyl-dihydro-furan-2-on | 412.31 | 412,0/ 414,0 |
| 87 | 5-Biphenyl-4-yl-3-(2-chlor-phenylamino)-5-methyl-dihydro-furan-2-on | 377.87 | 378,1 |
| 88 | 5-Biphenyl-4-yl-3-(2-chlor-4-fluor-phenylamino)-5-methyl-dihydro-furan-2-on | 395.86 | 396,1 |
| 90 | 5-Biphenyl-4-yl-3-(4-brom-5-phenyl-pyrazol-3-ylamino)-5-methyl-dihydro-furan-2-on | 488.38 | 490,1 |

### Allgemeine Arbeitsvorschrift 2

Unter Rühren wurden bei Raumtemperatur 1 Moläquivalent der jeweiligen Aminkomponente und 1,5 Moläquivalente Glyoxylsäure-Monohydrat in 10 ml Acetonitril gelöst, mit 1 Moläquivalent Trifluoressigsäure und anschließend mit 1,5 bis 3 Moläquivalenten der jeweiligen Alkenkomponente versetzt. Der Verlauf der Reaktion wurde durch Dünnschichtchromatographie verfolgt (Laufmittelsystem Diethylether / Hexan, 1:1) und ist nach zwei bis zwölf Stunden beendet (DC-Kontrolle). Der Reaktionsansatz wurde mit einem Überschuß an gesättigter, wäßriger Natriumhydrogencarbonat-Lösung versetzt und die wäßrige Phase dreimal mit Diethylether extrahiert. Die organische Phase wurde mit Wasser neutral gewaschen, über Magnesiumsulfat getrocknet, abfiltriert, mit Diethylether gewaschen und eingeengt. Die Produkte wurden direkt durch Ausfällen als Hydrochlorid bzw. nach Kieselgel-Chromatographie (Kieselgel 60) mit Ether/Diisopropylether- oder Ether/Hexan-Gemischen wechselnder Zusammensetzung isoliert.

Die Charakterisierung der isolierten Produkte erfolgte durch ¹H-NMR-Spektroskopie und ESI-Massenspektrometrie.

Die nach der allgemeinen Arbeitsvorschrift 2 hergestellten γ-Lactonverbindungen sind in der nachfolgenden Tabelle 2 widergegeben:

**Tabelle 2:**

| **Beispiel** | **Name** |
|---|---|
| 91 | 3-(3,5-Dichlorphenylamino)-5-methyl-5-phenyl-dihydrofuran-2-on Hydrochlorid |
| 92 | 3-(3,5-Dichlorphenylamino)-5-methyl-5-o-tolyl-dihydrofuran-2-on |
| 93 | 3-(3,5-Dichlorphenylamino)-5-(4-fluorphenyl)-5-methyl-dihydrofuran-2-on Hydrochlorid |
| 94 | 5-(2-Chlorphenyl)-3-(3,5-dichlorphenylamino)-5-methyl-dihydrofuran-2-on |
| 95 | 5-(4-Chlorphenyl)-3-(3,5-dichlorphenylamino)-5-methyl-dihydrofuran-2-on |
| 96 | 5-(3-Bromphenyl)-3-(3,5-dichlorphenylamino)-5-methyl-dihydrofuran-2-on Hydrochlorid |
| 97 | 5-(4-Bromphenyl)-3-(3,5-dichlorphenylamino)-5-methyl-dihydrofuran-2-on Hydrochlorid |
| 98 | 3-(3,5-Dichlorphenylamino)-5-(4-iodphenyl)-5-methyl-dihydrofuran-2-on Hydrochlorid |
| 99 | 3-(3,5-Dichlorphenylamino)-5-(2-methoxyphenyl)-5-methyl-dihydrofuran-2-on |
| 100 | 3-(3,5-Dichlorphenylamino)-5-(3-methoxyphenyl)-5-methyl-dihydrofuran-2-on Hydrochlorid |
| 101 | 3-(3,5-Dichlorphenylamino)-5-(4-methoxyphenyl)-5-methyl-dihydrofuran-2-on |
| 102 | 3-(3,5-Dichlorphenylamino)-5-(2,4-dimethoxyphenyl)-5-methyl-dihydrofuran-2-on |
| 103 | 3-(3,5-Dichlorphenylamino)-5-(2,5-dimethoxyphenyl)-5-methyl-dihydrofuran-2-on |
| 104 | 3-(3,5-Dichlorphenylamino)-5-(3,5-dimethoxyphenyl)-5-methyl-dihydrofuran-2-on |
| 105 | 5-(Biphenyl-4-yl-)3-(3,5-dichlorphenylamino)-5-methyl-dihydrofuran-2-on |
| 106 | 3-(3,5-Dichlorphenylamino)-5-ethyl-5-phenyl-dihydrofuran-2-on |
| 107 | 3-(3,5-Dichlorphenylamino)-5-phenyl-5-n-propyl-dihydrofuran-2-on |
| 108 | 5-n-Butyl-3-(3,5-dichlorphenylamino)-5-phenyl-dihydrofuran-2-on |
| 109 | 3-(3,5-Dichlorphenylamino)-7a-phenyl-hexahydrobenzofuran |
| 110 | 3-(3,5-Dichlorphenylamino)-7a-(3-methoxy-phenyl)-hexahydrobenzofuran-2-on |
| 111 | 3-(3,5-Dichlorphenylamino)-8a-(3-methoxy-phenyl)-octahydrocyclohepta[b]furan-2-on |

### Allgemeine Arbeitsvorschrift 3:

Die Umsetzungen unter Mikrowellen-Bestrahlung wurden in einer Labor-Mikrowelle vom Typ MLS ETHOS 600 der Firma MLS-GmbH (D-88299 Leutkirch, Auenweg 37, Deutschland) durchgeführt.

Zur Synthese wurden 1 Moläquivalent der jeweiligen Aminokomponente, 1,5 Moläquivalente Glyoxalsäure-Monohydrat und 1,5 bis 3-Moläquivalente der jeweiligen Alkenkomponente in Acetonitril in ein druckstabiles Teflongefäß gegeben, das mit einem Teflondeckel verschlossen und in eine Sicherungshalterung zum Ablassen von Überdruck eingespannt wurde. Die Kontrolle der Reaktionstemperatur erfolgte in einem zweiten Teflongefäß, das ebenfalls Acetonitril enthielt. Über eine interne Glasfaseroptik, die durch ein Quarzrohr in das zweite Teflongefäß führte, wurde die Reaktionstemperatur von einem externen Steuerungscomputer kontrolliert und nachgeregelt.

Das Reaktionsgemisch wurde in der Mikrowelle innerhalb einer Minute auf 55°C erhitzt und fünf Minuten bei dieser Temperatur belassen. Nach dem Abkühlen wurde das Gefäß in Eiswasser gestellt, vorsichtig geöffnet und einrotiert. Es wurde ein dunkelbraunes Öl erhalten.
Die so erhaltenen Rohprodukte wurden durch Kieselgel-Chromatographie (Kieselgel 60) mit Ether/Diisopropylether- oder Ether/Hexan-Gemischen unterschiedlicher Zusammensetzung gereinigt und anschließend durch Fällen als Hydrochloride isoliert.

Die Charakterisierung der isolierten Produkte erfolgte durch ¹H-NMR-Spektroskopie und ESI-Massenspektrometrie.

### Pharmakologische Untersuchungen

Untersuchungen zur Rezeptorbindung

Die Untersuchungen zur Bestimmung der Affinität der erfindungsgemäßen Verbindungen gemäß den Beispielen 12, 13, 25 und 108 zur Glycin-Bindungsstelle des NMDA-Rezeptorkanals wurden wie obenstehend beschrieben durchgeführt.

Die Affinität Glycin-Bindungsstelle des NMDA-Rezeptorkanals wurde als IC₅₀ (Konzentration mit 50 % Verdrängung des radioaktiven Liganden aus seiner spezifischen Bindung) nach dem Massenwirkungsgesetz mittels nichtlinearer Regression berechnet und ist in der nachstehenden Tabelle 3, nach Umrechnung (nach der Cheng-Prussoff-Gleichung (Y. Cheng, W.H. Prusoff, 1973, Biochem. Pharmacol., Vol. 22, pp. 3099-3108)) als Ki-Wert angegeben.

**Tabelle 3:**

| Beispiel | Glycin Bindungsstelle des NMDA-Rezeptorkanals | |
|---|---|---|
| | Ki (µM) | % Hemmung (10µM) |
| 12 | 0,8 | 80 |
| 13 | 0,8 | 70 |
| 25 | 1,3 | 71 |
| 108 | nicht bestimmt | 62 |

## Patentansprüche

1. Substituierte γ-Lactonverbindungen der allgemeinen Formel I, 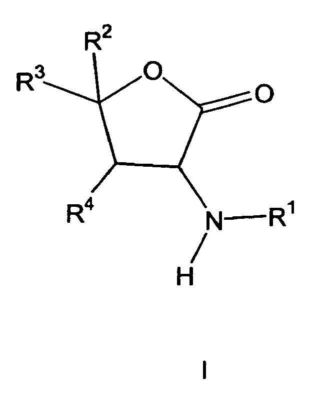 worin
R¹ für einen Aryl- oder Heteroaryl-Rest steht, der mit wenigstens einem Substituenten ausgewählt aus der Gruppe bestehend aus F, Cl, Br, I, NH₂, SH, OH, CF₃, CN, NO₂, OR⁵, SR⁵, NR⁶R⁷ und unsubstituiertem oder wenigstens einfach mit F, Cl, Br, I, NH₂, SH, OH, CF₃, CN oder NO₂ substituiertem C₁₋₆-Alkyl, C₁₋₆ Alkoxy, C₂₋₈-Alkenyl, C₂₋₈-Alkinyl, Phenyl, Phenoxy oder Benzyloxy substituiert sein kann;
R² für einen gesättigten, verzweigten oder unverzweigten aliphatischen C₁₋₁₀-Rest, der wenigstens einfach mit einem Substituenten ausgewählt aus der Gruppe bestehend aus F, Cl, Br, I, NH₂, SH und OH substituiert sein kann,
oder für einen zumindest teilweise ungesättigten, verzweigten oder unverzweigten aliphatischen C₂₋₁₀-Rest, der wenigstens einfach mit einem Substituenten ausgewählt aus der Gruppe bestehend aus F, Cl, Br, I, NH₂, SH und OH substituiert sein kann, steht;
R³ für einen Aryl-Rest steht, der mit wenigstens einem Substituenten ausgewählt aus der Gruppe bestehend aus F, Cl, Br, I, NH₂, SH, OH, CF₃, CN, NO₂, OR⁵, SR⁵, NR⁶R⁷ und unsubstituiertem oder wenigstens einfach mit F, Cl, Br, I, NH₂, SH, OH, CF₃, CN oder NO₂ substituiertem C₁₋₆-Alkyl, C₁₋₆-Alkoxy, C₂₋₈-Alkenyl, C₂₋₈-Alkinyl, Phenyl, Phenoxy oder Benzyloxy substituiert sein kann;
R⁴ für H steht,
oder
R³ und R⁴ zusammen für einen gesättigten oder wenigstens einfach ungesättigten aliphatischen C₃₋₇-Rest stehen, der wenigstens einfach mit einem Substituenten ausgewählt aus der Gruppe bestehend aus F, Cl, Br, I, NH₂, SH und OH substituiert sein kann,
mit der Maßgabe, daß der Rest R² in diesem Fall für einen Aryl-Rest, der mit wenigstens einem Substituenten ausgewählt aus der Gruppe bestehend aus F, Cl, Br, I, NH₂, SH, OH, CF₃, CN, NO₂, OR⁵, SR⁵, NR⁶R⁷ und unsubstituiertem oder wenigstens einfach mit F, Cl, Br, I, NH₂, SH, OH, CF₃, CN oder NO₂ substituiertem C₁₋₆-Alkyl, C₁₋₆-Alkoxy, C₂₋₈-Alkenyl, C₂₋₈-Alkinyl, Phenyl, Phenoxy oder Benzyloxy substituiert sein kann;
für einen gesättigten, verzweigten oder unverzweigten aliphatischen C₁₋₁₀-Rest,
der wenigstens einfach mit einem Substituenten ausgewählt aus der Gruppe bestehend aus F, Cl, Br, I, NH₂, SH und OH substituiert sein kann,
oder für einen zumindest teilweise ungesättigten, verzweigten oder unverzweigten aliphatischen C₂₋₁₀-Rest, der wenigstens einfach mit einem Substituenten ausgewählt aus der Gruppe bestehend aus F, Cl, Br, I, NH₂, SH und OH substituiert sein kann, steht,
R⁵ für einen C₁₋₁₀-Alkyl-Rest, für einen unsubstituierten Aryl- oder Heteroaryl-Rest oder für einen unsubstituierten, über eine C₁₋₃-Alkylen-Gruppe gebundenen Aryl- oder Heteroaryl-Rest steht und
R⁶ und R⁷, gleich oder verschieden, für H, einen C₁₋₁₀-Alkyl-Rest, für einen unsubstituierten Aryl- oder Heteroaryl-Rest oder für einen unsubstituierten, über eine C₁₋₃-Alkylen-Gruppe gebundenen Aryl- oder Heteroaryl-Rest stehen,
in Form ihrer Racemate, Diastereomere oder Enantiomere in Form ihrer Base oder eines entsprechenden physiologisch verträglichen Salzes,
wobei die Verbindung der allgemeinen Formel I, worin R¹ für einen 2-,4-,6-Trichlorphenyl-Rest, R² für einen Methyl-Rest, R³ für einen Phenyl-Rest und R⁴ für H steht, ausgenommen ist.

2. Substituierte γ-Lactonverbindungen gemäß Anspruch 1, **dadurch gekennzeichnet, daß**
R¹ für einen Aryl-Rest steht, der mit wenigstens einem Substituenten ausgewählt aus der Gruppe bestehend aus F, Cl, Br, I, NH₂, SH, OH, CF₃, CN, NO₂, OR⁵, SR⁵, NR⁶R⁷ und unsubstituiertem oder wenigstens einfach mit F, Cl, Br, I, NH₂, SH, OH, CF₃, CN oder NO₂ substituiertem C₁₋₆-Alkyl, C₁₋₆-Alkoxy, C₂₋₈-Alkenyl, C₂₋₈-Alkinyl, Phenyl, Phenoxy oder Benzyloxy substituiert sein kann.

3. Substituierte γ-Lactonverbindungen gemäß Anspruch 1 oder 2, **dadurch gekennzeichnet, daß**
R² für einen verzweigten oder unverzweigten C₁₋₆-Alkyl-Rest steht, der wenigstens einfach mit einem Substituenten ausgewählt aus der Gruppe bestehend aus F, Cl, Br, I, NH₂, SH und OH substituiert sein kann.

4. Substituierte γ-Lactonverbindungen gemäß einem der Ansprüche 1 bis 3, **dadurch gekennzeichnet, daß**
R³ für einen Aryl-Rest steht, der mit wenigstens einem Substituenten ausgewählt aus der Gruppe bestehend aus F, CI, Br, I, NH₂, SH, OH, CF₃, CN, NO₂, OR⁵, SR⁵, NR⁶R⁷ und unsubstituiertem oder wenigstens einfach mit F, CI, Br, I, NH₂, SH, OH, CF₃, CN oder NO₂ substituiertem C₁₋₆-Alkyl, C₁₋₆-Alkoxy, C₂₋₈-Alkenyl, C₂₋₈₋Alkinyl, Phenyl, Phenoxy oder Benzyloxy substituiert sein kann, und R⁴ für H steht.

5. Substituierte γ-Lactonverbindungen gemäß einem oder mehreren der Ansprüche 1 bis 4:
3-(2-Chlor-4-fluor-phenylamino)-5-(4-fluor-phenyl)-5-methyl-dihydro-furan-2-on,
5-Methyl-3-(4-phenoxy-phenylamino)-5-phenyl-dihydro-furan-2-on,
3-(2-Chlor-phenylamino)-5-(4-fluor-phenyl)-5-methyl-dihydro-furan-2-on,
3-(4-Chlor-2-methyl-phenylamino)-5-methyl-5-phenyl-dihydro-furan-2-on,
3-(2,4-Dichlor-phenylamino)-5-(4-fluor-phenyl)-5-methyl-dihydro-furan-2-on,
3-(4-Chlor-3-trifluormethyl-phenylamino)-5-methyl-5-phenyl-dihydro-furan-2-on,
3-(2,3-Dichlor-phenylamino)-5-(4-fluor-phenyl)-5-methyl-dihydro-furan-2-on,
3-(4-lod-phenylamino)-5-methyl-5-phenyl-dihydro-furan-2-on,
3-(4-Chlor-2-fluor-phenylamino)-5-(4-fluor-phenyl)-5-methyl-dihydro-furan-2-on,
3-(2-Chlor-4-methyl-phenylamino)-5-methyl-5-phenyl-dihydro-furan-2-on,
3-(2-Chlor-4-methyl-phenylamino)-5-(4-fluor-phenyl)-5-methyl-dihydro-furan-2-on,
3-(3,5-Dichlor-phenylamino)-5-methyl-5-phenyl-dihydro-furan-2-on,
3-(3,5-Dichlor-phenylamino)-5-(4-fluor-phenyl)-5-methyl-dihydro-furan-2-on,
3-(4-Brom-2-chlor-phenylamino)-5-(4-fluor-phenyl)-5-methyl-dihydro-furan-2-on,
4-(5-Methyl-2-oxo-5-phenyl-tetrahydro-furan-3-ylamino)-benzonitril,
5-(4-Chlor-phenyl)-3-(4-iod-phenylamino)-5-methyl-dihydro-furan-2-on,
5-(4-Chlor-phenyl)-3-(2,4-dichlor-phenylamino)-5-methyl-dihydro-furan-2-on,
5-(4-Chlor-phenyl)-3-(2-chlor-phenylamino)-5-methyl-dihydro-furan-2-on,
3-(4-Chlor-2-methyl-phenylamino)-5-(4-chlor-phenyl)-5-methyl-dihydro-furan-2-on
3-(2-Chlor-4-fluor-phenylamino)-5-(4-chlor-phenyl)-5-methyl-dihydro-furan-2-on,
3-(4-Chlor-2-fluor-phenylamino)-5-(4-chlor-phenyl)-5-methyl-dihydro-furan-2-on,
3-(2-Chlor-4-methyl-phenylamino)-5-(4-chlor-phenyl)-5-methyl-dihydro-furan-2-on,
5-(4-Chlor-phenyl)-3-(2,3-dichlor-phenylamino)-5-methyl-dihydro-furan-2-on,
3-(4-Brom-2-chlor-phenylamino)-5-(4-chlor-phenyl)-5-methyl-dihydro-furan-2-on,
5-(4-Chlor-phenyl)-3-(3,5-dichlor-phenylamino)-5-methyl-dihydro-furan-2-on,
3-(3,5-Dibrom-pyridin-2-ylamino)-5-methyl-5-phenyl-dihydro-furan-2-on,
5-(4-Chlor-phenyl)-3-(3,5-dichlor-pyridin-2-ylamino)-5-methyl-dihydro-furan-2-on,
5-(4-Chlor-phenyl)-5-methyl-3-(5-nitro-pyridin-2-ylamino)-dihydro-furan-2-on,
3-(3-Chlor-2-methyl-phenylamino)-5-(4-iod-phenyl)-5-methyl-dihydro-furan-2-on,
5-(4-Brom-phenyl)-3-(4-chlor-phenylamino)-5-methyl-dihydro-furan-2-on,
5-(3-Chlor-phenyl)-3-(4-chlor-phenylamino)-5-methyl-dihydro-furan-2-on,
3-(4-Chlor-phenylamino)-5-(4-iod-phenyl)-5-methyl-dihydro-furan-2-on,
5-(4-Brom-phenyl)-3-(2-iod-phenylamino)-5-methyl-dihydro-furan-2-on,
5-(3-Chlor-phenyl)-3-(2-iod-phenylamino)-5-methyl-dihydro-furan-2-on,
5-(4-lod-phenyl)-3-(2-iod-phenylamino)-5-methyl-dihydro-furan-2-on,
3-(2,4-Difluor-phenylamino)-5-methyl-5-naphthalin-1-yl-dihydro-furan-2-on,
5-(4-Brom-phenyl)-3-(4-iod-phenylamino)-5-methyl-dihydro-furan-2-on,
5-(3-Chlor-phenyl)-3-(4-iod-phenylamino)-5-methyl-dihydro-furan-2-on,
3-(4-lod-phenylamino)-5-methyl-5-naphthalin-1-yl-dihydro-furan-2-on,
5-(4-Brom-phenyl)-3-(3,5-dichlor-phenylamino)-5-methyl-dihydro-furan-2-on,
5-(3-Chlor-phenyl)-3-(3,5-dichlor-phenylamino)-5-methyl-dihydro-furan-2-on,
3-(3,5-Dichlor-phenylamino)-5-(4-iod-phenyl)-5-methyl-dihydro-furan-2-on,
3-(3,5-Dichlor-phenylamino)-5-methyl-5-naphthalin-1-yl-dihydro-furan-2-on,
5-(3-Chlor-phenyl)-5-methyl-3-phenylamino-dihydro-furan-2-on,
3-(2-Brom-4-methyl-phenylamino)-5-(4-iod-phenyl)-5-methyl-dihydro-furan-2-on,
3-(2-Brom-4-methyl-phenylamino)-5-methyl-5-naphthalin-1-yl-dihydro-furan-2-on,
3-(5-Chlor-2-methyl-phenylamino)-5-methyl-5-(5,6,7,8-tetrahydro-naphthalin-2-yl)-dihydro-furan-2-on,
3-(4-Brom-2-fluor-phenylamino)-5-isopropyl-5-phenyl-dihydro-furan-2-on,
5-(2,5-Dimethoxy-phenyl)-5-methyl-3-(5-trifluormethyl-pyridin-2-ylamino)-dihydrofuran-2-on,
5-(3,5-Dimethoxy-phenyl)-5-methyl-3-(5-trifluormethyl-pyridin-2-ylamino)-dihydrofuran-2-on,
3-(3-Brom-5-methyl-pyridin-2-ylamino)-5-(2-methoxy-phenyl)-5-methyl-dihydrofuran-2-on,
3-(3-Brom-5-methyl-pyridin-2-ylamino)-5-(2,5-dimethoxy-phenyl)-5-methyl-dihydrofuran-2-on,
3-(3-Brom-5-methyl-pyridin-2-ylamino)-5-(3,5-dimethoxy-phenyl)-5-methyl-dihydrofuran-2-on,
3-(5-Brom-3-methyl-pyridin-2-ylamino)-5-(2-methoxy-phenyl)-5-methyl-dihydrofuran-2-on,
3-(2-Chlor-pyridin-3-ylamino)-5-(2-methoxy-phenyl)-5-methyl-dihydro-furan-2-on,
3-(5-Brom-pyridin-2-ylamino)-5-(2,5-dimethoxy-phenyl)-5-methyl-dihydro-furan-2-on,
3-(3-Chlor-5-trifluormethyl-pyridin-2-ylamino)-5-(2,5-dimethoxy-phenyl)-5-methyldihydro-furan-2-on,
5-(2-Methoxy-phenyl)-5-methyl-3-(pyridin-2-ylamino)-dihydro-furan-2-on,
3-[5-(3-Brom-phenyl)-5-methyl-2-oxo-tetrahydro-furan-3-ylamino]-5-methylsulfanylpyrazol-4-carbonitril,
3-[5-(2,5-Dimethoxy-phenyl)-5-methyl-2-oxo-tetrahydro-furan-3-ylamino]-pyrazol-4-carbonitril,
3-(4-Brom-pyrazol-3-ylamino)-5-(3,5-dimethoxy-phenyl)-5-methyl-dihydro-furan-2-on,
3-(4-Brom-5-phenyl-2H-pyrazol-3-ylamino)-5-(2-methoxy-phenyl)-5-methyl-dihydrofuran-2-on,
3-(8-Hydroxy-chinolin-2-ylamino)-5-(2-methoxy-phenyl)-5-methyl-dihydro-furan-2-on,
5-(2,5-Dimethoxy-phenyl)-3-(8-hydroxy-chinolin-2-ylamino)-5-methyl-dihydro-furan-2-on,
5-(2-Methoxy-phenyl)-5-methyl-3-(pyrazin-2-ylamino)-dihydro-furan-2-on,
5-(3-Brom-phenyl)-5-methyl-3-(4-methyl-pyrimidin-2-ylamino)-dihydro-furan-2-on,
5-(2-Methoxy-phenyl)-5-methyl-3-(pyrimidin-2-ylamino)-dihydro-furan-2-on,
3-(4-Chlor-3-trifluormethyl-phenylamino)-5-phenyl-5-propyl-dihydro-furan-2-on,
3-(2-Chlor-phenylamino)-5-phenyl-5-propyl-dihydro-furan-2-on,
3-(2-Chlor-4-fluor-phenylamino)-5-phenyl-5-propyl-dihydro-furan-2-on,
3-(4-Chlor-2-fluor-phenylamino)-5-phenyl-5-propyl-dihydro-furan-2-on,
3-(2-Chlor-4-methyl-phenylamino)-5-phenyl-5-propyl-dihydro-furan-2-on,
3-(4-Brom-5-phenyl-pyrazol-3-ylamino)-5-phenyl-5-propyl-dihydro-furan-2-on,
5-Methylsulfanyl-3-(2-oxo-5-phenyl-5-propyl-tetrahydro-furan-3-ylamino)-pyrazol-4-carbonitril,
3-(4-Brom-5-phenyl-pyrazol-3-ylamino)-5-butyl-5-phenyl-dihydro-furan-2-on,
3-(5-Butyl-2-oxo-5-phenyl-tetrahydro-furan-3-ylamino)-5-methylsulfanyl-pyrazol-4-carbonitril,
3-(5-Butyl-2-oxo-5-phenyl-tetrahydro-furan-3-ylamino)-pyrazol-4-carbonitril,
5-Butyl-3-(2-phenoxy-phenylamino)-5-phenyl-dihydro-furan-2-on,
5-Biphenyl-4-yl-3-(2,4-dichlor-phenylamino)-5-methyl-dihydro-furan-2-on,
5-Biphenyl-4-yl-3-(2-chlor-phenylamino)-5-methyl-dihyd ro-fu ran-2-on,
5-Biphenyl-4-yl-3-(2-chlor-4-fluor-phenylamino)-5-methyl-dihydro-furan-2-on,
5-Biphenyl-4-yl-3-(4-brom-5-phenyl-pyrazol-3-ylamino)-5-methyl-dihydro-furan-2-on,
3-(3,5-Dichlorphenylamino)-5-methyl-5-phenyl-dihydrofuran-2-on,
3-(3,5-Dichlorphenylamino)-5-methyl-5-o-tolyl-dihydrofuran-2-on,
3-(3,5-Dichlorphenytamino)-5-(4-fluorphenyl)-5-methyl-dihydrofuran-2-on,
5-(2-Chlorphenyl)-3-(3,5-dichlorphenylamino)-5-methyl-dihydrofuran-2-on,
5-(4-Chlorphenyl)-3-(3,5-dichlorphenylamino)-5-methyl-dihydrofuran-2-on,
5-(3-Bromphenyl)-3-(3,5-dichlorphenylamino)-5-methyl-dihydrofuran-2-on,
5-(4-Bromphenyl)-3-(3,5-dichlorphenylamino)-5-methyl-dihydrofuran-2-on,
3-(3,5-Dichlorphenylamino)-5-(4-iodphenyl)-5-methyl-dihydrofuran-2-on,
3-(3,5-Dichlorphenylamino)-5-(2-methoxyphenyl)-5-methyl-dihydrofuran-2-on,
3-(3,5-Dichlorphenylamino)-5-(3-methoxyphenyl)-5-methyl-dihydrofuran-2-on,
3-(3,5-Dichlorphenylamino)-5-(4-methoxyphenyl)-5-methyl-dihydrofuran-2-on,
3-(3,5-Dichlorphenylamino)-5-(2,4-dimethoxyphenyl)-5-methyl-dihydrofuran-2-on,
3-(3,5-Dichlorphenylamino)-5-(2,5-dimethoxyphenyl)-5-methyl-dihydrofuran-2-on,
3-(3,5-Dichlorphenylamino)-5-(3,5-dimethoxyphenyl)-5-methyl-dihydrofuran-2-on,
5-(Biphenyl-4-yl-)3-(3,5-dichlorphenylamino)-5-methyl-dihydrofuran-2-on,
3-(3,5-Dichlorphenylamino)-5-ethyl-5-phenyl-dihydrofuran-2-on,
3-(3,5-Dichlorphenylamino)-5-phenyl-5-n-propyl-dihydrofuran-2-on,
5-n-Butyl-3-(3,5-dichlorphenylamino)-5-phenyl-dihydrofuran-2-on,
3-(3,5-Dichlorphenylamino)-7a-phenyl-hexahydrobenzofuran,
3-(3,5-Dichlorphenylamino)-7a-(3-methoxy-phenyl)-hexahydrobenzofuran-2-on
und
3-(3,5-Dichlorphenylamino)-8a-(3-methoxy-phenyl)-octahydrocyclo-hepta[b]furan-2-on
sowie deren entsprechende physiologisch verträgliche Salze, vorzugsweise deren Hydrochloride.

6. Verfahren zur Herstellung substituierter γ-Lactonverbindungen gemäß einem der Ansprüche 1 bis 5, **dadurch gekennzeichnet, daß** man wenigstens eine Aminkomponente der allgemeinen Formel II, 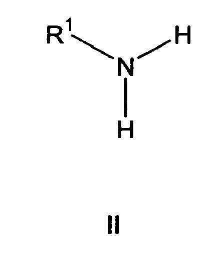 worin der Rest R¹ die Bedeutung gemäß den Ansprüchen 1 bis 5 hat, mit Glyoxalsäure und wenigstens einer Alkenkomponente der allgemeinen Formel III, 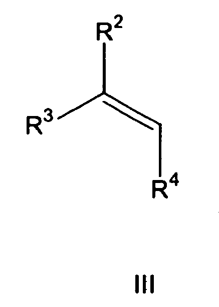 worin die Reste R² bis R⁴ die Bedeutung gemäß den Ansprüchen 1 bis 5 haben, in Gegenwart von zumindest einer anorganischen und/oder organischen Säure in einem organischen Lösungsmittel zu wenigstens einer Verbindung der allgemeinen Formel I gemäß den Ansprüchen 1 bis 5 umsetzt und diese gegebenenfalls nach üblichen Methoden reinigt und/oder gegebenenfalls nach üblichen Methoden isoliert.

7. Verfahren gemäß Anspruch 6, **dadurch gekennzeichnet, daß** die Glyoxalsäure in Form ihres Monohydrats oder in Form einer wäßrigen Lösung eingesetzt wird.

8. Verfahren gemäß Anspruch 6 oder 7, **dadurch gekennzeichnet, daß** als organische Säure Trifluoressigsäure eingesetzt wird.

9. Verfahren gemäß einem der Ansprüche 6 bis 8, **dadurch gekennzeichnet, daß** die Temperatur während der Umsetzung 0 bis 100 °C, vorzugsweise 15 bis 40 °C beträgt.

10. Verfahren gemäß einem der Ansprüche 6 bis 9, **dadurch gekennzeichnet, daß** die Umsetzungsdauer 0,25 bis 12 Stunden beträgt.

11. Verfahren zur Herstellung substituierter γ-Lactonverbindungen gemäß einem der Ansprüche 1 bis 5, **dadurch gekennzeichnet, daß** man wenigstens eine Aminkomponente der allgemeinen Formel II gemäß Anspruch 6 mit Glyoxalsäure und wenigstens einer Alkenkomponente der allgemeinen Formel III gemäß Anspruch 6 in einem organischen Lösungsmittel ggf. in Gegenwart zumindest einer anorganischen und/oder organischen Säure unter Mikrowellenbestrahlung oder unter Einwirkung von Ultraschall, vorzugsweise unter Mikrowellenbestrahlung, zu wenigstens einer Verbindung der allgemeinen Formel I gemäß den Ansprüchen 1 bis 5 umsetzt und diese gegebenenfalls nach üblichen Methoden reinigt und/oder gegebenenfalls nach üblichen Methoden isoliert.

12. Verfahren gemäß Anspruch 11, **dadurch gekennzeichnet, daß** die Temperatur während der Umsetzung 40 bis 70 °C, vorzugsweise 45 bis 60 °C beträgt.

13. Arzneimittel enthaltend wenigstens eine substituierte γ-Lactonverbindung gemäß einem der Ansprüche 1 bis 5 und gegebenenfalls physiologisch verträgliche Hilfsstoffe.

14. Arzneimittel gemäß Anspruch 13 zur Bekämpfung von Schmerz.

15. Arzneimittel gemäß Anspruch 14 zur Bekämpfung von chronischem Schmerz.

16. Arzneimittel gemäß Anspruch 14 zur Bekämpfung von neuropathischem Schmerz.

17. Arzneimittel gemäß Anspruch 13 zur Behandlung oder Prophylaxe von neurodegenerativen Erkrankungen, vorzugsweise von Morbus Alzheimer, Morbus Parkinson oder Morbus Huntington.

18. Arzneimittel gemäß Anspruch 13 zur Behandlung oder Prophylaxe von Schlaganfall.

19. Arzneimittel gemäß Anspruch 13 zur Behandlung oder Prophylaxe von cerebraler lschämie.

20. Arzneimittel gemäß Anspruch 13 zur Behandlung oder Prophylaxe von cerebralem Infarkt.

21. Arzneimittel gemäß Anspruch 13 zur Behandlung oder Prophylaxe von Himödem.

22. Arzneimittel gemäß Anspruch 13 zur Anxiolyse.

23. Arzneimittel gemäß Anspruch 13 zur Anästhesie.

24. Arzneimittel gemäß Anspruch 13 zur Behandlung oder Prophylaxe von Schizophrenie.

25. Arzneimittel gemäß Anspruch 13 zur Behandlung oder Prophylaxe von Psychosen bedingt durch erhöhten Aminosäurespiegel.

26. Arzneimittel gemäß Anspruch 13 zur Behandlung oder Prophylaxe von AIDS-Demenz.

27. Arzneimittel gemäß Anspruch 13 zur Behandlung oder Prophylaxe des Tourette-Syndroms.

28. Arzneimittel gemäß Anspruch 13 zur Behandlung oder Prophylaxe von inflammatorischen und/oder allergischen Reaktionen.

29. Arzneimittel gemäß Anspruch 13 zur Behandlung oder Prophylaxe von Depressionen.

30. Arzneimittel gemäß Anspruch 13 zur Behandlung oder Prophylaxe von seelischen Erkrankungen.

31. Arzneimittel gemäß Anspruch 13 zur Behandlung oder Prophylaxe von Epilepsie.

32. Arzneimittel gemäß Anspruch 13 zur Behandlung oder Prophylaxe von Haminkontinenz.

33. Arzneimittel gemäß Anspruch 13 zur Behandlung oder Prophylaxe von Juckreiz.

34. Arzneimittel gemäß Anspruch 13 zur Behandlung oder Prophylaxe von Tinnitus aurium.

35. Arzneimittel gemäß Anspruch 13 zur Behandlung oder Prophylaxe von Diarrhoe.

36. Verwendung wenigstens einer substituierten γ-Lactonverbindung gemäß einem der Ansprüche 1 bis 5 zur Herstellung eines Arzneimittels zur Bekämpfung von Schmerz, vorzugsweise von chronischem oder neuropathischem Schmerz.

37. Verwendung wenigstens einer substituierten γ-Lactonverbindung gemäß einem der Ansprüche 1 bis 5 zur Herstellung eines Arzneimittels zur Behandlung oder Prophylaxe von neurodegenerativen Erkrankungen, vorzugsweise von Morbus Alzheimer, Morbus Parkinson oder Morbus Huntington, zur Behandlung oder Prophylaxe von Migräne, Schlaganfall, cerebraler Ischämie, cerebralem Infarkt, Himödem, Schizophrenie, Psychosen bedingt durch erhöhten Aminosäurespiegel, AIDS-Demenz, Tourette-Syndrom, inflammatorischen und/oder allergischen Reaktionen, Depressionen, seelischen Erkrankungen, Epilepsie, Haminkontinenz, Juckreiz, Tinnitus aurium, Diarrhoe, zur Anxiolyse oder zur Anästhesie.

## Claims

1. Substituted γ-lactone compounds of the general formula I, 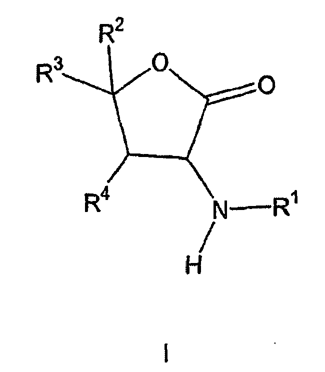 in which
R¹ denotes an aryl or heteroaryl residue, which may be substituted with at least one substituent selected from the group consisting of F, Cl, Br, I, NH₂, SH, OH, CF₃, CN, NO₂, OR⁵, SR⁵, NR⁶R⁷ and C₁₋₆ alkyl, C₁₋₆ alkoxy, C₂₋₈ alkenyl, C₂₋₈ alkynyl, phenyl, phenoxy or benzyloxy which are unsubstituted or at least mono- substituted with F, Cl, Br, I, NH₂, SH, OH, CF₃, CN or NO₂;
R² denotes a saturated, branched or unbranched aliphatic C₁₋₁₀ residue, which may be at least monosubstituted with a substituent selected from the group consisting of F, Cl, Br, I, NH₂, SH and OH,
or an at least partially unsaturated, branched or unbranched aliphatic C₂₋₁₀ residue, which may be at least monosubstituted with a substituent selected from the group consisting of F, Cl, Br, I, NH₂, SH and OH;
R³ denotes an aryl residue, which may be substituted with at least one substituent selected from the group consisting of F, Cl, Br, I, NH₂, SH, OH, CF₃, CN, NO₂, OR⁵, SR⁵, NR⁶R⁷ and C₁₋₆ alkyl, C₁₋₆ alkoxy, C₂₋₈ alkenyl, C₂₋₈ alkynyl, phenyl, phenoxy or benzyloxy which are unsubstituted or at least mono-substituted with F, Cl, Br, I, NH₂, SH, OH, CF₃, CN or NO₂;
R⁴ denotes H,
or
R³ and R⁴ together denote a saturated or at least mono-unsaturated aliphatic C₃₋₇ residue, which may be at least monosubstituted with a substituent selected from the group consisting of F, Cl, Br, I, NH₂, SH and OH,
with the proviso that the residue R² in this case denotes an aryl residue, which may be substituted with at least one substituent selected from the group consisting of F, Cl, Br, I, NH₂, SH, OH, CF₃, CN, NO₂, OR⁵, SR⁵, NR⁶R⁷ and C₁₋₆ alkyl, C₁₋₆ alkoxy, C₂₋₈ alkenyl, C₂₋₈ alkynyl, phenyl, phenoxy or benzyloxy which are unsubstituted or at least mono-substituted with F, Cl, Br, I, NH₂, SH, OH, CF₃, CN or NO₂;
a saturated, branched or unbranched aliphatic C₁₋₁₀ residue, which may be at least monosubstituted with a substituent selected from the group consisting of F, Cl, Br, I, NH₂, SH and OH,
or an at least partially unsaturated, branched or unbranched aliphatic C₂₋₁₀ residue, which may be at least monosubstituted with a substituent selected from the group consisting of F, Cl, Br, I, NH₂, SH and OH,
R⁵ denotes a C₁₋₁₀ alkyl residue, an unsubstituted aryl or heteroaryl residue or denotes an unsubstituted aryl or heteroaryl residue attached via a C₁₋₃ alkylene group and
R⁶ and R⁷, identical or different, denote H, a C₁₋₁₀ alkyl residue, an unsubstituted aryl or heteroaryl residue or denote an unsubstituted aryl or heteroaryl residue attached via a C₁₋₃ alkylene group,
in the form of the racemates, diastereomers or enantiomers thereof in the form of the base thereof or of a corresponding physiologically acceptable salt,
wherein the compounds of the general formula I, in which R¹ denotes a 2-,4-,6-trichlorophenyl residue, R² a methyl residue, R³ a phenyl residue and R⁴ denotes H, are excepted.

2. Substituted γ-lactone compounds according to claim 1, **characterised in that**
R¹ denotes an aryl or heteroaryl residue, which may be substituted with at least one substituent selected from the group consisting of F, Cl, Br, I, NH₂, SH, OH, CF₃, CN, NO₂, OR⁵, SR⁵, NR⁶R⁷ and C₁₋₆ alkyl, C₁₋₆ alkoxy, C₂₋₈ alkenyl, C₂₋₈ alkynyl, phenyl, phenoxy or benzyloxy which are unsubstituted or at least mono- substituted with F, Cl, Br, I, NH₂, SH, OH, CF₃, CN or NO₂.

3. Substituted γ-lactone compounds according to claim 1 or claim 2, **characterised in that**
R² denotes a branched or unbranched C₁₋₆ alkyl residue, which may be at least monosubstituted with a substituent selected from the group consisting of F, Cl, Br, I, NH₂, SH and OH.

4. Substituted γ-lactone compounds according to any one of claims 1 to 3, **characterised in that**
R³ denotes an aryl residue, which may be substituted with at least one substituent selected from the group consisting of F, Cl, Br, I, NH₂, SH, OH, CF₃, CN, NO₂, OR⁵, SR⁵, NR⁶R⁷ and C₁₋₆ alkyl, C₁₋₆ alkoxy, C₂₋₈ alkenyl, C₂₋₈ alkynyl, phenyl, phenoxy or benzyloxy which are unsubstituted or at least mono-substituted with F, Cl, Br, I, NH₂, SH, OH, CF₃, CN or NO₂, and R⁴ denotes H.

5. Substituted γ-lactone compounds according to one or more of claims 1 to 4:
3-(2-Chloro-4-fluoro-phenylamino)-5-(4-fluoro-phenyl)-5-methyl-dihydro-furan-2-one,
5-Methyl-3-(4-phenoxy-phenylamino)-5-phenyl-dihydro-furan-2-one,
3-(2-Chloro-phenylamino)-5-(4-fluoro-phenyl)-5-methyl-dihydro-furan-2-one,
3-(4-Chloro-2-methyl-phenylamino)-5-methyl-5-phenyl-dihydro-furan-2-one,
3-(2,4-Dichloro-phenylamino)-5-(4-fluoro-phenyl)-5-methyl-dihydro-furan-2-one,
3-(4-Chloro-3-trifluoromethyl-phenylamino)-5-methyl-5-phenyl-dihydro-furan-2-one,
3-(2,3-Dichloro-phenylamino)-5-(4-fluoro-phenyl)-5-methyl-dihydro-furan-2-one,
3-(4-Iodo-phenylamino)-5-methyl-5-phenyl-dihydro-furan-2-one,
3-(4-Chloro-2-fluoro-phenylamino)-5-(4-fluoro-phenyl)-5-methyl-dihydro-furan-2-one,
3-(2-Chloro-4-methyl-phenylamino)-5-methyl-5-phenyl-dihydro-furan-2-one,
3-(2-Chloro-4-methyl-phenylamino)-5-(4-fluoro-phenyl)-5-methyl-dihydro-furan-2-one,
3-(3,5-Dichloro-phenylamino)-5-methyl-5-phenyl-dihydro-furan-2-one,
3-(3,5-Dichloro-phenylamino)-5-(4-fluoro-phenyl)-5-methyl-dihydro-furan-2-one,
3-(4-Bromo-2-chloro-phenylamino)-5-(4-fluoro-phenyl)-5-methyl-dihydro-furan-2-one,
4-(5-Methyl-2-oxo-5-phenyl-tetrahydro-furan-3-ylamino)-benzonitrile,
5-(4-Chloro-phenyl)-3-(4-iodo-phenylamino)-5-methyl-dihydro-furan-2-one,
5-(4-Chloro-phenyl)-3-(2,4-dichloro-phenylamino)-5-methyl-dihydro-furan-2-one,
5-(4-Chloro-phenyl)-3-(2-chloro-phenylamino)-5-methyl-dihydro-furan-2-one,
3-(4-Chloro-2-methyl-phenylamino)-5-(4-chloro-phenyl)-5-methyl-dihydro-furan-2-one
3-(2-Chloro-4-fluoro-phenylamino)-5-(4-chloro-phenyl)-5-methyl-dihydro-furan-2-one,
3-(4-Chloro-2-fluoro-phenylamino)-5-(4-chloro-phenyl)-5-methyl-dihydro-furan-2-one,
3-(2-Chloro-4-methyl-phenylamino)-5-(4-chloro-phenyl)-5-methyl-dihydro-furan-2-one,
5-(4-Chloro-phenyl)-3-(2,3-dichloro-phenylamino)-5-methyl-dihydro-furan-2-one,
3-(4-Bromo-2-chloro-phenylamino)-5-(4-chloro-phenyl)-5-methyl-dihydro-furan-2-one,
5-(4-Chloro-phenyl)-3-(3,5-dichloro-phenylamino)-5-methyl-dihydro-furan-2-one,
3-(3,5-Dibromo-pyridin-2-ylamino)-5-methyl-5-phenyl-dihydro-furan-2-one,
5-(4-Chloro-phenyl)-3-(3,5-dichloro-pyridin-2-ylamino)-5-methyl-dihydro-furan-2-one,
5-(4-Chloro-phenyl)-5-methyl-3-(5-nitro-pyridin-2-ylamino)-dihydro-furan-2-one,
3-(3-Chloro-2-methyl-phenylamino)-5-(4-iodo-phenyl)-5-methyl-dihydro-furan-2-one,
5-(4-Bromo-phenyl)-3-(4-chloro-phenylamino)-5-methyl-dihydro-furan-2-one,
5-(3-Chloro-phenyl)-3-(4-chloro-phenylamino)-5-methyl-dihydro-furan-2-one,
3-(4-chloro-phenylamino)-5-(4-iodo-phenyl)-5-methyl-dihydro-furan-2-one,
5-(4-Bromo-phenyl)-3-(2-iodo-phenylamino)-5-methyl-dihydro-furan-2-one,
5-(3-Chloro-phenyl)-3-(2-iodo-phenylamino)-5-methyl-dihydro-furan-2-one,
5-(4-Iodo-phenyl)-3-(2-iodo-phenylamino)-5-methyl-dihydro-furan-2-one,
3-(2,4-Difluoro-phenylamino)-5-methyl-5-naphthalen-1-yl-dihydro-furan-2-one,
5-(4-Bromo-phenyl)-3-(4-iodo-phenylamino)-5-methyl-dihydro-furan-2-one,
5-(3-Chloro-phenyl)-3-(4-iodo-phenylamino)-5-methyl-dihydro-furan-2-one,
3-(4-Iodo-phenylamino)-5-methyl-5-naphthalen-1-yl-dihydro-furan-2-one,
5-(4-Bromo-phenyl)-3-(3,5-dichloro-phenylamino)-5-methyl-dihydro-furan-2-one,
5-(3-Chloro-phenyl)-3-(3,5-dichloro-phenylamino)-5-methyl-dihydro-furan-2-one,
3-(3,5-Dichloro-phenylamino)-5-(4-iodo-phenyl)-5-methyl-dihydro-furan-2-one,
3-(3,5-Dichloro-phenylamino)-5-methyl-5-naphthalen-1-yl-dihydro-furan-2-one,
5-(3-Chloro-phenyl)-5-methyl-3-phenylamino-dihydro-furan-2-one,
3-(2-Bromo-4-methyl-phenylamino)-5-(4-iodo-phenyl)-5-methyl-dihydro-furan-2-one,
3-(2-Bromo-4-methyl-phenylamino)-5-methyl-5-naphthalen-1-yl-dihydro-furan-2-one,
3-(5-Chloro-2-methyl-phenylamino)-5-methyl-5-(5,6,7,8-tetrahydro-naphthalen-2-yl)-dihydro-furan-2-one,
3-(4-Bromo-2-fluoro-phenylamino)-5-isopropyl-5-phenyl-dihydro-furan-2-one,
5-(2,5-Dimethoxy-phenyl)-5-methyl-3-(5-trifluoromethyl-pyridin-2-ylamino)-dihydro-furan-2-one,
5-(3,5-Dimethoxy-phenyl)-5-methyl-3-(5-trifluoromethyl-pyridin-2-ylamino)-dihydro-furan-2-one,
3-(3-Bromo-5-methyl-pyridin-2-ylamino)-5-(2-methoxy-phenyl)-5-methyl-dihydro-furan-2-one,
3-(3-Bromo-5-methyl-pyridin-2-ylamino)-5-(2,5-dimethoxy-phenyl)-5-methyl-dihydro-furan-2-one,
3-(3-Bromo-5-methyl-pyridin-2-ylamino)-5-(3,5-dimethoxy-phenyl)-5-methyl-dihydro-furan-2-one,
3-(5-Bromo-3-methyl-pyridin-2-ylamino)-5-(2-methoxy-phenyl)-5-methyl-dihydro-furan-2-one,
3-(2-Chloro-pyridin-3-ylamino)-5-(2-methoxy-phenyl)-5-methyl-dihydro-furan-2-one,
3-(5-Bromo-pyridin-2-ylamino)-5-(2,5-dimethoxy-phenyl)-5-methyl-dihydro-furan-2-one,
3-(3-Chloro-5-trifluoromethyl-pyridin-2-ylamino)-5-(2,5-dimethoxy-phenyl)-5-methyl-dihydro-furan-2-one,
5-(2-Methoxy-phenyl)-5-methyl-3-(pyridin-2-ylamino)-dihydro-furan-2-one,
3-[5-(3-Bromo-phenyl)-5-methyl-2-oxo-tetrahydro-furan-3-ylamino]-5-methylsulfanyl-pyrazole-4-carbonitrile,
3-[5-(2,5-Dimethoxy-phenyl)-5-methyl-2-oxo-tetrahydro-furan-3-ylamino]-pyrazole-4-carbonitrile,
3-(4-Bromo-pyrazol-3-ylamino)-5-(3,5-dimethoxy-phenyl)-5-methyl-dihydro-furan-2-one,
3-(4-Bromo-5-phenyl-2H-pyrazol-3-ylamino)-5-(2-methoxy-phenyl)-5-methyl-dihydro-furan-2-one,
3-(8-Hydroxy-quinolin-2-ylamino)-5-(2-methoxy-phenyl)-5-methyl-dihydro-furan-2-one,
5-(2,5-Dimethoxy-phenyl)-3-(8-hydroxy-quinolin-2-ylamino)-5-methyl-dihydro-furan-2-one,
5-(2-Methoxy-phenyl)-5-methyl-3-(pyrazin-2-ylamino)-dihydro-furan-2-one,
5-(3-Bromo-phenyl)-5-methyl-3-(4-methyl-pyrimidin-2-ylamino)-dihydro-furan-2-one,
5-(2-Methoxy-phenyl)-5-methyl-3-(pyrimidin-2-ylamino)-dihydro-furan-2-one,
3-(4-Chloro-3-trifluoromethyl-phenylamino)-5-phenyl-5-propyl-dihydro-furan-2-one,
3-(2-Chloro-phenylamino)-5-phenyl-5-propyl-dihydro-furan-2-one,
3-(2-Chloro-4-fluoro-phenylamino)-5-phenyl-5-propyl-dihydro-furan-2-one,
3-(4-Chloro-2-fluoro-phenylamino)-5-phenyl-5-propyl-dihydro-furan-2-one,
3-(2-Chloro-4-methyl-phenylamino)-5-phenyl-5-propyl-dihydro-furan-2-one,
3-(4-Bromo-5-phenyl-pyrazol-3-ylamino)-5-phenyl-5-propyl-dihydro-furan-2-one,
5-Methylsulfanyl-3-(2-oxo-5-phenyl-5-propyl-tetrahydro-furan-3-ylamino)-pyrazole-4-carbonitrile,
3-(4-Bromo-5-phenyl-pyrazol-3-ylamino)-5-butyl-5-phenyl-dihydro-furan-2-one,
3-(5-Butyl-2-oxo-5-phenyl-tetrahydro-furan-3-ylamino)-5-methylsulfanyl-pyrazole-4-carbonitrile,
3-(5-Butyl-2-oxo-5-phenyl-tetrahydro-furan-3-ylamino)-pyrazole-4-carbonitrile,
5-Butyl-3-(2-phenoxy-phenylamino)-5-phenyl-dihydro-furan-2-one,
5-Biphenyl-4-yl-3-(2,4-dichloro-phenylamino)-5-methyl-dihydro-furan-2-one,
5-Biphenyl-4-yl-3-(2-chloro-phenylamino)-5-methyl-dihydro-furan-2-one,
5-Biphenyl-4-yl-3-(2-chloro-4-fluoro-phenylamino)-5-methyl-dihydro-furan-2-one,
5-Biphenyl-4-yl-3-(4-bromo-5-phenyl-pyrazol-3-ylamino)-5-methyl-dihydro-furan-2-one,
3-(3,5-Dichlorophenylamino)-5-methyl-5-phenyl-dihydrofuran-2-one,
3-(3,5-Dichlorophenylamino)-5-methyl-5-o-tolyl-dihydrofuran-2-one,
3-(3,5-Dichlorophenylamino)-5-(4-fluorophenyl)-5-methyl-dihydrofuran-2-one,
5-(2-Chlorophenyl)-3-(3,5-dichlorophenylamino)-5-methyl-dihydrofuran-2-one,
5-(4-Chlorophenyl)-3-(3,5-dichlorophenylamino)-5-methyl-dihydrofuran-2-one,
5-(3-Bromophenyl)-3-(3,5-dichlorophenylamino)-5-methyl-dihydrofuran-2-one,
5-(4-Bromophenyl)-3-(3,5-dichlorophenylamino)-5-methyl-dihydrofuran-2-one,
3-(3,5-Dichlorophenylamino)-5-(4-iodophenyl)-5-methyl-dihydrofuran-2-one,
3-(3,5-Dichlorophenylamino)-5-(2-methoxyphenyl)-5-methyl-dihydrofuran-2-one,
3-(3,5-Dichlorophenylamino)-5-(3-methoxyphenyl)-5-methyl-dihydrofuran-2-one,
3-(3,5-Dichlorophenylamino)-5-(4-methoxyphenyl)-5-methyl-dihydrofuran-2-one,
3-(3,5-Dichlorophenylamino)-5-(2,4-dimethoxyphenyl)-5-methyl-dihydrofuran-2-one,
3-(3,5-Dichlorophenylamino)-5-(2,5-dimethoxyphenyl)-5-methyl-dihydrofuran-2-one,
3-(3,5-Dichlorophenylamino)-5-(3,5-dimethoxyphenyl)-5-methyl-dihydrofuran-2-one,
5-(Biphenyl-4-yl-)3-(3,5-dichlorophenylamino)-5-methyl-dihydrofuran-2-one,
3-(3,5-Dichlorophenylamino)-5-ethyl-5-phenyl-dihydrofuran-2-one,
3-(3,5-Dichlorophenylamino)-5-phenyl-5-n-propyl-dihydrofuran-2-one,
5-n-Butyl-3-(3,5-dichlorophenylamino)-5-phenyl-dihydrofuran-2-one,
3-(3,5-Dichlorophenylamino)-7a-phenyl-hexahydrobenzofuran,
3-(3,5-Dichlorophenylamino)-7a-(3-methoxy-phenyl)-hexahydrobenzofuran-2-one
and
3-(3,5-Dichlorophenylamino)-8a-(3-methoxy-phenyl)-octahydrocyclo-hepta[b]furan-2-one
and the corresponding physiologically acceptable salts thereof, preferably the hydrochlorides thereof.

6. A process for the production of substituted γ-lactone compounds according to any one of claims 1 to 5, **characterised in that** at least one amine component of the general formula II, 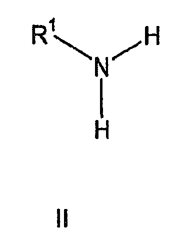 in which the residue R¹ has the meaning according to claims 1 to 5, is reacted with glyoxalic acid and at least one alkene component of the general formula III, 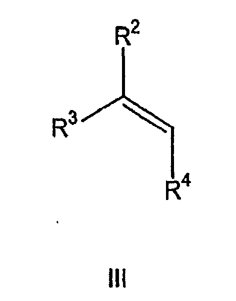 in which the residues R² to R⁴ have the meaning according to claims 1 to 5, in the presence of at least one inorganic and/or organic acid in an organic solvent to yield at least one compound of the general formula I according to claims 1 to 5 and this is optionally purified using conventional methods and/or optionally isolated using conventional methods.

7. A process according to claim 6, **characterised in that** the glyoxalic acid is used in the form of the monohydrate thereof or in form of an aqueous solution.

8. A process according to claim 6 or claim 7, **characterised in that** trifluoroacetic acid is used as the organic acid.

9. A process according to any one of claims 6 to 8, **characterised in that** the temperature during the reaction is 0 to 100°C, preferably 15 to 40°C.

10. A process according to any one of claims 6 to 9, **characterised in that** the duration of the reaction is 0.25 to 12 hours.

11. A process for the production of substituted γ-lactone compounds according to any one of claims 1 to 5, **characterised in that** at least one amine component of the general formula II according to claim 6 is reacted with glyoxalic acid and at least one alkene component of the general formula III according to claim 6 in an organic solvent, optionally in the presence at least one inorganic and/or organic acid with microwave irradiation or with exposure to ultrasound, preferably with microwave irradiation, to yield at least one compound of the general formula I according to claims 1 to 5 and this is optionally purified using conventional methods and/or optionally isolated using conventional methods.

12. A process according to claim 11, **characterised in that** the temperature during the reaction is 40 to 70°C, preferably 45 to 60°C.

13. A pharmaceutical preparation containing at least one substituted γ-lactone compound according to any one of claims 1 to 5 and optionally physiologically acceptable auxiliary substances.

14. A pharmaceutical preparation according to claim 13 for combatting pain.

15. A pharmaceutical preparation according to claim 14 for combatting chronic pain.

16. A pharmaceutical preparation according to claim 14 for combatting neuropathic pain.

17. A pharmaceutical preparation according to claim 13 for the treatment or prevention of neurodegenerative diseases, preferably of Alzheimer's disease, Parkinson's disease or Huntington's chorea.

18. A pharmaceutical preparation according to claim 13 for the treatment or prevention of stroke.

19. A pharmaceutical preparation according to claim 13 for the treatment or prevention of cerebral ischaemia.

20. A pharmaceutical preparation according to claim 13 for the treatment or prevention of cerebral infarct.

21. A pharmaceutical preparation according to claim 13 for the treatment or prevention of cerebral oedema.

22. A pharmaceutical preparation according to claim 13 for anxiolysis.

23. A pharmaceutical preparation according to claim 13 for anaesthesia.

24. A pharmaceutical preparation according to claim 13 for the treatment or prevention of schizophrenia.

25. A pharmaceutical preparation according to claim 13 for the treatment or prevention of psychoses brought about by elevated amino acid levels.

26. A pharmaceutical preparation according to claim 13 for the treatment or prevention of AIDS dementia.

27. A pharmaceutical preparation according to claim 13 for the treatment or prevention of Tourette's syndrome.

28. A pharmaceutical preparation according to claim 13 for the treatment or prevention of inflammatory and/or allergic reactions.

29. A pharmaceutical preparation according to claim 13 for the treatment or prevention of depression.

30. A pharmaceutical preparation according to claim 13 for the treatment or prevention of mental health conditions.

31. A pharmaceutical preparation according to claim 13 for the treatment or prevention of epilepsy.

32. A pharmaceutical preparation according to claim 13 for the treatment or prevention of urinary incontinence.

33. A pharmaceutical preparation according to claim 13 for the treatment or prevention of pruritus.

34. A pharmaceutical preparation according to claim 13 for the treatment or prevention of tinnitus.

35. A pharmaceutical preparation according to claim 13 for the treatment or prevention of diarrhoea.

36. Use of at least one substituted γ-lactone compound according to any one of claims 1 to 5 for the production of a pharmaceutical preparation for combatting pain, preferably chronic or neuropathic pain.

37. Use of at least one substituted γ-lactone compound according to any one of claims 1 to 5 for the production of a pharmaceutical preparation for the treatment or prevention of neurodegenerative diseases, preferably of Alzheimer's disease, Parkinson's disease or Huntington's chorea, for the treatment or prevention of migraine, stroke, cerebral ischaemia, cerebral infarct, cerebral oedema, schizophrenia, psychoses brought about by elevated amino acid levels, AIDS dementia, Tourette's syndrome, inflammatory and/or allergic reactions, depression, mental health conditions, epilepsy, urinary incontinence, pruritus, tinnitus, diarrhoea, for anxiolysis or for anaesthesia.

## Revendications

1. Composés substitués de γ-lactone de formule générale I, 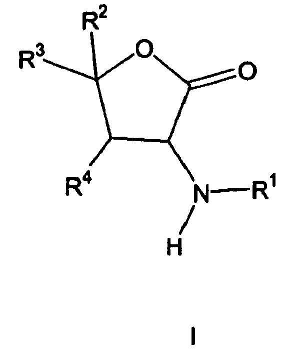 dans laquelle
R¹ représente un radical aryle ou hétéroaryle, qui peut être substitué par au moins un substituant du groupe constitué par F, Cl, Br, I, NH₂, SH, OH, CF₃, CN, NO₂, OR⁵, SR⁵, NR⁶R⁷ et par un radical alkyle en C₁ à C₆, alcoxy en C₁ à C₆, alcényle en C₂ à C₈, alcynyle en C₂ à C₈, phényle, phénoxy ou benzyloxy, non substitué ou au moins monosubstitué par F, Cl, Br, I, NH₂, SH, OH, CF₃, CN ou NO₂ ;
R² représente un radical aliphatique en C₁ à C₁₀ saturé, ramifié ou non ramifié, qui peut être au moins monosubstitué par un substituant choisi dans le groupe constitué par F, Cl, Br, I, NH₂, SH et OH,
ou un radical aliphatique en C₂ à C₁₀ au moins partiellement insaturé, ramifié ou non ramifié qui peut être au moins monosubstitué par un substituant choisi dans le groupe constitué par F, Cl, Br, I, NH₂, SH et OH ;
R³ représente un radical aryle, qui peut être substitué par au moins un substituant choisi dans le groupe constitué par F, Cl, Br, I, NH₂, SH, OH, CF₃, CN, NO₂, OR⁵, SR⁵, NR⁶R⁷ et par un radical alkyle en C₁ à C₆, alcoxy en C₁ à C₆, alcényle en C₂ à C₈, alcynyle en C₂ à C₈, phényle, phénoxy ou benzyloxy, non substitué ou au moins monosubstitué par F, Cl, Br, I, NH₂, SH, OH, CF₃, CN ou NO₂ ;
R⁴ représente H, ou
R³ et R⁴ représentent ensemble un radical aliphatique en C₃ à C₇ saturé ou au moins monoinsaturé, qui peut être au moins monosubstitué par un substituant choisi dans le groupe constitué par F, Cl, Br, I, NH₂, SH et OH,
à condition que le radical R² représente dans ce cas un radical aryle, qui peut être substitué par au moins un substituant choisi dans le groupe constitué par F, Cl, Br, I, NH₂, SH, OH, CF₃, CN, NO₂, OR⁵, SR⁵, NR⁶R⁷ et par un radical alkyle en C₁ à C₆, alcoxy en C₁ à C₆, alcényle en C₂ à C₈, alcynyle en C₂ à C₈, phényle, phénoxy ou benzyloxy, non substitué ou au moins monosubstitué par F, Cl, Br, I, NH₂, SH, OH, CF₃, CN ou NO₂ ;
un radical aliphatique en C₁ à C₁₀ saturé, ramifié ou non ramifié qui peut être au moins monosubstitué par un substitué choisi dans le groupe constitué par F, Cl, Br, I, NH₂, SH et OH,
ou un radical aliphatique en C₂ à C₁₀ au moins partiellement insaturé, ramifié ou non ramifié qui peut être au moins monosubstitué par un substituant choisi dans le groupe constitué par F, Cl, Br, I, NH₂, SH et OH ;
R⁵ représente un radical alkyle en C₁ à C₁₀, un radical aryle ou hétéroaryle non substitué ou un radical aryle ou hétéroaryle non substitué, lié via un groupe alkylène en C₁ à C₃ et
R⁶ et R⁷ sont identiques ou différents et représentent H, un radical alkyle en C₁ à C₁₀, un radical aryle ou hétéroaryle non substitué ou un radical aryle ou hétéroaryle non substitué, lié via un groupe alkylène en C₁ à C₃,
sous forme de leurs racémates, diastéréo-isomères ou énantiomères sous forme de leurs bases ou d'un sel physiologiquement acceptable correspondant,
le composé de formule générale I, dans lequel R¹ représente un radical 2-4-6-trichlorophényle, R² représente un radical méthyle, R³ représente un radical phényle et R⁴ représente H étant exclu.

2. Composés substitués de γ-lactone selon la revendication 1, **caractérisés en ce que**
R¹ représente un radical aryle, qui peut être substitué par au moins un substituant choisi dans le groupe constitué par F, Cl, Br, I, NH₂, SH, OH, CF₃, CN, NO₂, OR⁵, SR⁵, NR⁶R⁷ et par un radical alkyle en C₁ à C₆, alcoxy en C₁ à C₆, alcényle en C₂ à C₈, alcynyle en C₂ à C₈, phényle, phénoxy ou benzyloxy, non substitué ou au moins monosubstitué par F, Cl, Br, I, NH₂, SH, OH, CF₃, CN ou NO₂.

3. Composés substitués de γ-lactone selon la revendication 1 ou 2, **caractérisés en ce que**
R² représente un radical alkyle en C₁ à C₆ ramifié ou non ramifié, qui peut être au moins monosubstitué par un substituant choisi dans le groupe constitué par F, C1, Br, I, NH₂, SH et OH.

4. Composés substitués de γ-lactone selon l'une quelconque des revendications 1 à 3, **caractérisé en ce que**
R³ représente un radical aryle, qui peut être substitué par au moins un substituant choisi dans le groupe constitué par F, Cl, Br, I, NH₂, SH, OH, CF₃, CN, NO₂, OR⁵, SR⁵, NR⁶R⁷ et par un radical alkyle en C₁ à C₆, alcoxy en C₁ à C₆, alcényle en C₂ à C₈, alcynyle en C₂ à C₈, phényle, phénoxy ou benzyloxy, non substitué ou au moins monosubstitué par F, Cl, Br, I, NH₂, SH, OH, CF₃, CN ou NO₂ R⁴ représente H.

5. Composés substitués de γ-lactone selon l'une ou plusieurs des revendications 1 à 4 :
3-(2-chloro-4-fluoro-phénylamino)-5-(4-fluoro-phényl)-5-méthyl-dihydro-furann-2-one,
5-méthyl-3-(4-phénoxy-phénylamino)-5-phényl-dihydro-furann-2-one,
3-(2-chloro-phénylamino)-5-(4-fluoro-phényl)-5-méthyl-dihydro-furann-2-one,
3-(4-chloro-2-méthyl-phénylamino)-5-méthyl-5-phényl-dihydro-furann-2-one,
3-(2,4-dichloro-phénylamino)-5-(4-fluoro-phényl)-5-méthyl-dihydro-furann-2-one,
3-(4-chloro-3-trifluorométhyl-phénylamino)-5-méthyl-5-phényl-dihydro-furann-2-one,
3-(2,3-dichloro-phénylamino)-5-(4-fluoro-phényl)-5-méthyl-dihydro-furann-2-one,
3-(4-iodo-phénylamino)-5-méthyl-5-phényl-dihydro-furann-2-one,
3-(4-chloro-2-fluoro-phénylamino)-5-(4-fluoro-phényl)-5-méthyl-dihydro-furann-2-one,
3-(2-chloro-4-méthyl-phénylamino)-5-méthyl-5-phényl-dihydro-furann-2-one,
3-(2-chloro-4-méthyl-phénylamino)-5-(4-fluoro-phényl)-5-méthyl-dihydro-furann-2-one,
3-(3,5-dichloro-phénylamino)-5-méthyl-5-phényl-dihydro-furann-2-one,
3-(3,5-dichloro-phénylamino)-5-(4-fluoro-phényl)-5-méthyl-dihydro-furann-2-one,
3-(4-bromo-2-chloro-phénylamino)-5-(4-fluoro-phényl)-5-méthyl-dihydro-furann-2-one,
4-(5-méthyl-2-oxo-5-phényl-tétrahydro-furann-3-ylamino)-benzonitrile,
5-(4-chloro-phényl)-3-(4-iodo-phénylamino)-5-méthyl-dihydro-furann-2-one,
5-(4-chloro-phényl)-3-(2,4-dichloro-phénylamino)-5-méthyl-dihydro-furann-2-one,
5-(4-chloro-phényl)-3-(2-chloro-phénylamino)-5-méthyl-dihydro-furann-2-one,
3-(4-chloro-2-méthyl-phénylamino)-5-(4-chloro-phényl)-5-méthyl-dihydro-furann-2-one
3-(2-chloro-4-fluoro-phénylamino)-5-(4-chloro-phényl)-5-méthyl-dihydro-furann-2-one,
3-(4-chloro-2-fluoro-phénylamino)-5-(4-chloro-phényl)-5-méthyl-dihydro-furann-2-one,
3-(2-chloro-4-méthyl-phénylamino)-5-(4-chloro-phényl)-5-méthyl-dihydro-furann-2-one,
5-(4-chloro-phényl)-3-(2,3-dichloro-phénylamino)-5-méthyl-dihydro-furann-2-one,
3-(4-bromo-2-chloro-phénylamino)-5-(4-chloro-phényl)-5-méthyl-dihydro-furann-2-one,
5-(4-chloro-phényl)-3-(3,5-dichloro-phénylamino)-5-méthyl-dihydro-furann-2-one,
3-(3,5-dibromo-pyridin-2-ylamino)-5-méthyl-5-phényl-dihydro-furann-2-one,
5-(4-chloro-phényl)-3-(3,5-dichloro-pyridin-2-ylamino)-5-méthyl-dihydro-furann-2-one,
5-(4-chloro-phényl)-5-méthyl-3-(5-nitro-pyridin-2-ylamino)-dihydro-furann-2-one,
3-(3-chloro-2-méthyl-phénylamino)-5-(4-iodo-phényl)-5-méthyl-dihydro-furann-2-one,
5-(4-bromo-phényl)-3-(4-chloro-phénylamino)-5-méthyl-dihydro-furann-2-one,
5-(3-chloro-phényl)-3-(4-chloro-phénylamino)-5-méthyl-dihydro-furann-2-one,
3-(4-chloro-phénylamino)-5-(4-iodo-phényl)-5-méthyl-dihydro-furann-2-one,
5-(4-bromo-phényl)-3-(2-iodo-phénylamino)-5-méthyl-dihydro-furann-2-one,
5-(3-chloro-phényl)-3-(2-iodo-phénylamino)-5-méthyl-dihydro-furann-2-one,
5-(4-iodo-phényl)-3-(2-iodo-phénylamino)-5-méthyl-dihydro-furann-2-one,
3-(2,4-difluoro-phénylamino)-5-méthyl-5-naphtalén-1-yl-dihydro-furann-2-one,
5-(4-bromo-phényl)-3-(4-iodo-phénylamino)-5-méthyl-dihydro-furann-2-one,
5-(3-chloro-phényl)-3-(4-iodo-phénylamino)-5-méthyl-dihydro-furann-2-one,
3-(4-iodo-phénylamino)-5-méthyl-5-naphtalén-1-yl-dihydro-furann-2-one,
5-(4-bromo-phényl)-3-(3,5-dichloro-phénylamino)-5-méthyl-dihydro-furann-2-one,
5-(3-chloro-phényl)-3-(3,5-dichloro-phénylamino)-5-méthyl-dihydro-furann-2-one,
3-(3,5-dichloro-phénylamino)-5-(4-iodo-phényl)-5-méthyl-dihydro-furann-2-one,
3-(3,5-dichloro-phénylamino)-5-méthyl-5-naphtalén-1-yl-dihydro-furann-2-one,
5-(3-chloro-phényl)-5-méthyl-3-phénylamino-dihydro-furann-2-one,
3-(2-bromo-4-méthyl-phénylamino)-5-(4-iodo-phényl)-5-méthyl-dihydro-furann-2-one,
3-(2-bromo-4-méthyl-phénylamino)-5-méthyl-5-naphtalén-1-yl-dihydro-furann-2-one,
3-(5-chloro-2-méthyl-phénylamino)-5-méthyl-5-(5,6,7,8-tétrahydro-naphtalén-2-yl)-dihydro-furann-2-one,
3-(4-bromo-2-fluoro-phénylamino)-5-isopropyl-5-phényl-dihydro-furann-2-one,
5-(2,5-diméthoxy-phényl)-5-méthyl-3-(5-trifluorométhyl-pyridin-2-ylamino)-dihydro-furann-2-one,
5-(3,5-diméthoxy-phényl)-5-méthyl-3-(5-trifluorométhyl-pyridin-2-ylamino)-dihydro-furann-2-one,
3-(3-bromo-5-méthyl-pyridin-2-ylamino)-5-(2-méthoxy-phényl)-5-méthyl-dihydro-furann-2-one,
3-(3-bromo-5-méthyl-pyridin-2-ylamino)-5-(2,5-diméthoxy-phényl)-5-méthyl-dihydro-furann-2-one,
3-(3-bromo-5-méthyl-pyridin-2-ylamino)-5-(3,5-diméthoxy-phényl)-5-méthyl-dihydro-furann-2-one,
3-(5-bromo-3-méthyl-pyridin-2-ylamino)-5-(2-méthoxy-phényl)-5-méthyl-dihydro-furann-2-one,
3-(2-chloro-pyridin-3-ylamino)-5-(2-méthoxy-phényl)-5-méthyl-dihydro-furann-2-one,
3-(5-bromo-pyridin-2-ylamino)-5-(2,5-diméthoxy-phényl)-5-méthyl-dihydro-furann-2-one,
3-(3-chloro-5-trifluorométhyl-pyridin-2-ylamino)-5-(2,5-diméthoxy-phényl)-5-méthyl-dihydro-furann-2-one,
5-(2-méthoxy-phényl)-5-méthyl-3-(pyridin-2-ylamino)-dihydro-furann-2-one,
3-[5-(3-bromo-phényl)-5-méthyl-2-oxo-tétrahydro-furann-3-ylamino]-5-méthylsulfanyl-pyrazole-4-carbonitrile,
3-[5-(2,5-diméthoxy-phényl)-5-méthyl-2-oxo-tétrahydro-furann-3-ylamino]-pyrazole-4-carbonitrile,
3-(4-bromo-pyrazol-3-ylamino)-5-(3,5-diméthoxy-phényl)-5-méthyl-dihydro-furann-2-one,
3-(4-bromo-5-phényl-2H-pyrazol-3-ylamino)-5-(2-méthoxy-phényl)-5-méthyl-dihydro-furann-2-one,
3-(8-hydroxy-quinoléin-2-ylamino)-5-(2-méthoxy-phényl)-5-méthyl-dihydro-furann-2-one,
5-(2,5-diméthoxy-phényl)-3-(8-hydroxy-quinoléin-2-ylamino)-5-méthyl-dihydro-furann-2-one,
5-(2-méthoxy-phényl)-5-méthyl-3-(pyrazin-2-ylamino)-dihydro-furann-2-one,
5-(3-bromo-phényl)-5-méthyl-3-(4-méthyl-pyrimidin-2-ylamino)-dihydro-furann-2-one,
5-(2-méthoxy-phényl)-5-méthyl-3-(pyrimidin-2-ylamino)-dihydro-furann-2-one,
3-(4-chloro-3-trifluorométhyl-phénylamino)-5-phényl-5-propyl-dihydro-furann-2-one,
3-(2-chloro-phénylamino)-5-phényl-5-propyl-dihydro-furann-2-one,
3-(2-chloro-4-fluoro-phénylamino)-5-phényl-5-propyl-dihydro-furann-2-one,
3-(4-chloro-2-fluoro-phénylamino)-5-phényl-5-propyl-dihydro-furann-2-one,
3-(2-chloro-4-méthyl-phénylamino)-5-phényl-5-propyl-dihydro-furann-2-one,
3-(4-bromo-5-phényl-pyrazol-3-ylamino)-5-phényl-5-propyl-dihydro-furann-2-one,
5-méthylsulfanyl-3-(2-oxo-5-phényl-5-propyl-tétrahydro-furann-3-ylamino)-pyrazole-4-carbonitrile,
3-(4-bromo-5-phényl-pyrazol-3-ylamino)-5-butyl-5-phényl-dihydro-furann-2-one,
3-(5-butyl-2-oxo-5-phényl-tétrahydro-furann-3-ylamino)-5-méthylsulfanyl-pyrazole-4-carbonitrile,
3-(5-butyl-2-oxo-5-phényl-tétrahydro-furann-3-ylamino)-pyrazole-4-carbonitrile,
5-butyl-3-(2-phénoxy-phénylamino)-5-phényl-dihydro-furann-2-one,
5-biphényl-4-yl-3-(2,4-dichloro-phénylamino)-5-méthyl-dihydro-furann-2-one,
5-biphényl-4-yl-3-(2-chloro-phénylamino)-5-méthyl-dihydro-furann-2-one,
5-biphényl-4-yl-3-(2-chloro-4-fluoro-phénylamino)-5-méthyl-dihydro-furann-2-one,
5-biphényl-4-yl-3-(4-bromo-5-phényl-pyrazol-3-ylamino)-5-méthyl-dihydro-furann-2-one,
3-(3,5-dichlorophénylamino)-5-méthyl-5-phényl-dihydrofurann-2-one,
3-(3,5-dichlorophénylamino)-5-méthyl-5-o-tolyl-dihydrofurann-2-one,
3-(3,5-dichlorophénylamino)-5-(4-fluorophényl)-5-méthyl-dihydrofurann-2-one,
5-(2-chlorophényl)-3-(3,5-dichlorophénylamino)-5-méthyl-dihydrofurann-2-one,
5-(4-chlorophényl)-3-(3,5-dichlorophénylamino)-5-méthyl-dihydrofurann-2-one,
5-(3-bromophényl)-3-(3,5-dichlorophénylamino)-5-méthyl-dihydrofurann-2-one,
5-(4-bromophényl)-3-(3,5-dichlorophénylamino)-5-méthyl-dihydrofurann-2-one,
3-(3,5-dichlorophénylamino)-5-(4-iodophényl)-5-méthyl-dihydrofurann-2-one,
3-(3,5-dichlorophénylamino)-5-(2-méthoxyphényl)-5-méthyl-dihydrofurann-2-one,
3-(3,5-dichlorophénylamino)-5-(3-méthoxyphényl)-5-méthyl-dihydrofurann-2-one,
3-(3,5-dichlorophénylamino)-5-(4-méthoxyphényl)-5-méthyl-dihydrofurann-2-one,
3-(3,5-dichlorophénylamino)-5-(2,4-diméthoxyphényl)-5-méthyl-dihydrofurann-2-one,
3-(3,5-dichlorophénylamino)-5-(2,5-diméthoxyphényl)-5-méthyl-dihydrofurann-2-one,
3-(3,5-dichlorophénylamino)-5-(3,5-diméthox-yphényl)-5-méthyl-dihydrofurann-2-one,
5-(biphényl-4-yl-)3-(3,5-dichlorophénylamino)-5-méthyl-dihydrofurann-2-one,
3-(3,5-dichlorophénylamino)-5-éthyl-5-phényl-dihydrofurann-2-one,
3-(3,5-dichlorophénylamino)-5-phényl-5-n-propyl-dihydrofurann-2-one,
5-n-butyl-3-(3,5-dichlorophénylamino)-5-phényl-dihydrofurann-2-one,
3-(3,5-dichlorophénylamino)-7a-phényl-hexahydrobenzofuranne,
3-(3,5-dichlorophénylamino)-7a-(3-méthoxy-phényl)-hexahydrobenzofurann-2-one et
3-(3,5-dichlorophénylamino)-8a-(3-méthoxy-phényl)-octahydrocyclo-hepta[b]furann-2-one
ainsi que leurs sels physiologiquement acceptables correspondants, de préférence leurs chlorhydrates.

6. Procédé pour la préparation de composés substitués de γ-lactone selon l'une quelconque des revendications 1 à 5, **caractérisé en ce qu'**on transforme au moins un composant amine de formule générale II, 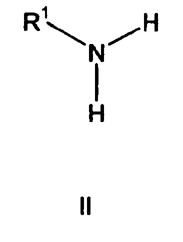 dans laquelle le radical R¹ a la signification selon les revendications 1 à 5, avec de l'acide glyoxalique et au moins un composé alcène de formule générale III, 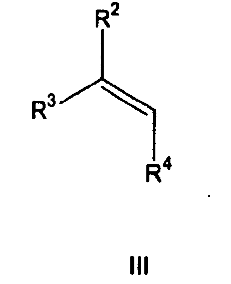 dans laquelle les radicaux R² à R⁴ ont la signification selon les revendications 1 à 5, en présence d'au moins un acide inorganique et/ou organique dans un solvant organique en au moins un composé de formule générale I selon les revendications 1 à 5 et on purifie le cas échéant celui-ci selon des procédés usuels et/ou isole celui-ci le cas échéant selon des procédés usuels.

7. Procédé selon la revendication 6, **caractérisé en ce que** l'acide glyoxalique est utilisé sous forme de son monohydrate ou sous forme d'une solution aqueuse.

8. Procédé selon la revendication 6 ou 7, **caractérisé en ce qu'**on utilise de l'acide trifluoroacétique comme acide organique.

9. Procédé selon l'une quelconque des revendications 6 à 8, **caractérisé en ce que** la température pendant la transformation est de 0 à 100°C, de préférence de 15 à 40°C.

10. Procédé selon l'une quelconque des revendications 6 à 9, **caractérisé en ce que** la durée de la transformation est de 0,25 à 12 heures.

11. Procédé pour la préparation de composés substitués de γ-lactone selon l'une quelconque des revendications 1 à 5, **caractérisé en ce qu'**on transforme au moins un composant amine de formule générale II selon la revendication 6 avec de l'acide glyoxalique et au moins un composant alcène de formule générale III selon la revendication 6 dans un solvant organique, le cas échéant en présence d'au moins un acide inorganique et/ou organique, sous une irradiation par des micro-ondes ou sous l'effet d'ultrasons, de préférence sous une irradiation aux micro-ondes, en au moins un composé de formule générale I selon les revendications 1 à 5 et on purifie celui-ci le cas échéant selon des procédés usuels et/ou on isole celui-ci le cas échéant selon des procédés usuels.

12. Procédé selon la revendication 11, **caractérisé en ce que** la température pendant la transformation est de 40 à 70°C, de préférence de 45 à 60°C.

13. Médicament contenant au moins un composé substitué de γ-lactone selon l'une quelconque des revendications 1 à 5 et le cas échéant des adjuvants physiologiquement acceptables.

14. Médicament selon la revendication 13, destiné à lutter contre la douleur.

15. Médicament selon la revendication 14 pour lutter contre la douleur chronique.

16. Médicament selon la revendication 14 pour lutter contre la douleur neuropathique.

17. Médicament selon la revendication 13 pour le traitement ou la prophylaxie de maladies de neurodégénérescence, de préférence de la maladie d'Alzheimer, de Parkinson ou de Huntington.

18. Médicament selon la revendication 13 pour le traitement ou la prophylaxie d'une attaque.

19. Médicament selon la revendication 13 pour le traitement ou la prophylaxie de l'ischémie cérébrale.

20. Médicament selon la revendication 13 pour le traitement ou la prophylaxie de l'infarctus cérébral.

21. Médicament selon la revendication 13 pour le traitement ou la prophylaxie de l'oedème cérébral.

22. Médicament selon la revendication 13 pour l'anxiolyse.

23. Médicament selon la revendication 13 pour l'anesthésie.

24. Médicament selon la revendication 13 pour le traitement ou la prophylaxie de la schizophrénie.

25. Médicament selon la revendication 13 pour le traitement ou la prophylaxie de psychoses provoquées par un niveau élevé d'acides aminés.

26. Médicament selon la revendication 13 pour le traitement ou la prophylaxie de la démence liée au SIDA.

27. Médicament selon la revendication 13 pour le traitement ou la prophylaxie du syndrome de Tourette.

28. Médicament selon la revendication 13 pour le traitement ou la prophylaxie de réactions inflammatoires et/ou allergiques.

29. Médicament selon la revendication 13 pour le traitement ou la prophylaxie de dépressions.

30. Médicament selon la revendication 13 pour le traitement ou la prophylaxie de maladies psychiques.

31. Médicament selon la revendication 13 pour le traitement ou la prophylaxie de l'épilepsie.

32. Médicament selon la revendication 13 pour le traitement ou la prophylaxie de l'incontinence urinaire.

33. Médicament selon la revendication 13 pour le traitement ou la prophylaxie du prurit.

34. Médicament selon la revendication 13 pour le traitement ou la prophylaxie des acouphènes (Tinnitus aurium).

35. Médicament selon la revendication 13 pour le traitement ou la prophylaxie de la diarrhée.

36. Utilisation d'au moins un composé substitué de γ-lactone selon l'une quelconque des revendications 1 à 5 pour la préparation d'un médicament destiné à lutter contre la douleur, de préférence la douleur chronique ou la douleur neuropathique.

37. Utilisation d'au moins un composé substitué de γ-lactone selon l'une quelconque des revendications 1 à 5 pour la préparation d'un médicament destiné au traitement ou à la prophylaxie de maladies de neurodégénérescence, de préférence la maladie d'Alzheimer, de Parkinson ou de Huntington, pour le traitement ou la prophylaxie de la migraine, d'une attaque, d'une ischémie cérébrale, d'un infarctus cérébral, d'un oedème cérébral, de la schizophrénie, de psychoses provoquées par un niveau élevé d'acides aminés, de la démence liée au SIDA, du syndrome de Tourette, des réactions inflammatoires et/ou allergiques, des dépressions, des maladies psychiques, de l'épilepsie, de l'incontinence urinaire, du prurit, des acouphènes (Tinnitus aurium), de la diarrhée, pour l'anxiolyse ou pour l'anesthésie.
